# FASCICULE DE BREVET EUROPEEN

(11) **EP 1 390 467 B1**
(45) Date de publication et mention de la délivrance du brevet: **16.01.2008**
(21) Numéro de dépôt: 02735507.2
(22) Date de dépôt: 26.04.2002
(51) Int. Cl.: C12M 3/04, C12M 1/34

(54) **UTILISATION D' UN DISPOSITIF MINIATURE DE SÉPARATION ET D' ISOLEMENT D'OBJETS BIOLOGIQUES ET PROCÉDÉ DE SÉPARATION**
VERWENDUNG EINER MINIATURVORRICHTUNG ZUR TRENNUNG UND ISOLIERUNG BIOLOGISCHER OBJEKTE UND TRENNUNGSVERFAHREN
USE OF A MINIATURE DEVICE FOR SEPARATING AND ISOLATING OBJECTS AND SEPARATION METHOD

(30) Priorité: 27.04.2001 FR 0105706
(43) Date de publication de la demande: 25.02.2004
(73) Titulaire: COMMISSARIAT A L'ENERGIE ATOMIQUE, 75015 Paris (FR); Proteus, 30000 Nîmes (FR)
(72) Inventeur: CAILLAT, Patrice, F-38130 Echirolles (FR); FUCHS, Alexandra, F-38120 Saint-Egreve (FR); REVOL-CAVALIER, Frédéric, F-38180 Seyssins (FR); DUPRET, Daniel, F-30420 Sinsans - Calvisson (FR); LEFEVRE, Fabrice, F-30900 Nîmes (FR)
(74) Mandataire: Goulard, Sophie
(86) Numéro de dépôt international: PCT/FR2002/001459
(87) Numéro de publication internationale: WO 2002/088300

(56) Documents cités:
- WO-A-00/36145
- DE-A- 19 841 125
- FR-A- 2 781 886
- US-A- 6 113 768

## Description

La présente Invention est relative aux utilisations d'un dispositif miniature de séparation et d'isolement d'objets biologiques pour diverses applications dans le domaine de la biologie moléculaire, aux procédés de fabrication de puces à ADN ou à protéines notamment, ainsi qu'aux puces à ADN ou à protéines obtenues par la mise en oeuvre de ces procédés.

La biologie, et en particulier la génomique, connaît actuellement une révolution dans la façon de générer et de traiter les données de ses analyses. Alors que de plus en plus de données de séquences génétiques sont disponibles grâce aux grands projets de séquençages des organismes, les acteurs de la biologie, du monde médical et de l'industrie pharmaceutique cherchent à intégrer l'ensemble de ces données dans des analyses à grande échelle et multiparamétriques.

Le monde des microtechnologies, en particulier celui des microsystèmes, a les moyens de répondre à cette demande grâce à ses acquis en miniaturisation, en fonctionnalisation des surfaces, en microfluidique et en techniques de fabrication à grande échelle et à faible coût.

Actuellement, le mariage de ces deux mondes a donné lieu à des outils d'analyse multiparamétriques connus sous le nom de puces à ADN. Ces outils sont dédiés à l'analyse de macromolécules biologiques (protéines et principalement ADN).

Il existe un nombre important de recherches de part le monde dans des domaines très variés qui intègrent, via les microtechnologies, des protocoles biologiques à des dimensions de plus en plus réduites.

Par exemple, les plaques de microtitration sont passées d'un format standard de 96 puits à un format de 384 puis de 1536 puits au fur et à mesure des progrès de la robotique. L'utilisation de ces microtechniques de plus en plus miniaturisées permet de diminuer les volumes des réactifs utilisés et ainsi de réduire les coûts d'analyse.

Dans le cas particulier des puces à ADN, le principe d'analyse consiste à ordonner en matriçage X-Y des sondes nucléiques à des pas de plus en plus petits, de l'ordre de 20 µm.

De même, l'étape de traitement des échantillons biologiques subit la course à la réduction de taille avec l'intégration de plus en plus commune de réactions de polymérisation en chaîne (PCR) dans des puces à ADN ou de fonction de lyse des cellules.

C'est ainsi qu'il a déjà été proposé, notamment dans le brevet US 6,071,394, de séparer et fixer des cellules sur des puces électroniques par diélectrophorèse, les cellules en suspension dans un tampon approprié étant séparées en fonction de leurs propriétés diélectriques. Cependant cette technique ne permet pas de séparer et d'isoler des objets biologiques uniques pour des analyses individualisées.

La tendance actuelle qui passe par une réduction des volumes de réactifs utilisés a également pour conséquence l'utilisation de tests des analytes sur des réactifs biologiques fixés sur des surfaces solides, de façon à abandonner progressivement l'utilisation de tests en tubes avec des solutions homogènes.

C'est ainsi qu'il a déjà été proposé diverses solutions pour la fixation de molécules biologiques d'intérêt sur différents matériaux tels que le verre, le plastique ou le métal. Par exemple, pour la fixation des sondes nucléiques sur substrat, trois approches principales sont actuellement connues :
- l'utilisation d'un substrat en verre recouvert de poly-L-lysine qui est un polymère présentant une affinité avec les sondes nucléiques (Schena M. et al., science, 1995, 270 (5235), 467-470),
- l'utilisation de verre recouvert d'un silane fonctionnalisé, la sonde nucléique portant alors une fonction complémentaire de façon à former une liaison covalente avec le silane (O'Donnell M.J. et al., Anal. Chem., 1997, 69,2438-2443),
- l'utilisation d'électrodes métalliques, en or par exemple, permettant la copolymérisation d'un monomère simple et d'un monomère porteur de la sonde nucléique.

De même, dans le domaine de la protéomique, il a notamment été rapporté la fixation de peptides par l'intermédiaire de polymères conducteurs portant des fonctions pyrroles (polypyrroles), (Livache T. et al., Biosensors & Bioelectronics, 1998,13, 629-634).

Il est également possible de fixer des objets biologiques vivants tels que des bactéries ou des cellules eucaryotes sur des substrats solides par différentes techniques :
- greffage d'anticorps spécifiques de la cellule à fixer sur un substrat solide ; il existe en effet des anticorps contre les micro-organismes couramment utilisés en biologie moléculaire (bactéries, levures), tels que des anticorps anti-*E*. *coli* 1434 vendus par la société Fitzgerald Industries International, Inc., ainsi que des anticorps contre des récepteurs de surfaces (récepteurs CD) des cellules eucaryotes supérieures lymphoïdes,
- greffage de peptides spécifiques de certains types cellulaires sur le support (Holland J. et al., Biomaterials, 1996,17,2147-2156),
- fonctionnalisation non spécifique du support par des polymères permettant l'adhésion cellulaire (Aframian D.J. et al., Tissue Eng., 2000, 6 (3), 209-216).

Ces techniques sont intéressantes dans la mesure où elles génèrent la formation de liaisons spécifiques entre l'objet biologique à fixer et le support ; cependant elles nécessitent de nombreuses étapes de préparation et d'isolement avant que ceux-ci puissent enfin être fixés individuellement sur le support.

D'autre part, il a déjà été proposé, notamment dans les demandes internationales WO 00/09747, WO 00/34512 et 00/34514 des procédés d'identification de séquences polynucléotidiques et/ou des protéines correspondantes à partir d'un échantillon d'acides nucléiques. Cependant, ces procédés nécessitent la préparation individuelle des fragments d'ADN et par conséquent de nombreuses opérations de pipetage.

Les Inventeurs se sont donc fixés pour but de pourvoir à un dispositif miniature de séparation et d'isolement d'objets biologiques permettant d'effectuer différents types d'applications dans le domaine de la biologie moléculaire tout en conservant une approche matricielle à grand nombre de points dans laquelle chaque point contient un et un seul type ou catégorie d'objet biologique, mais dans lequel les opérations préalables de préparation, de séparation ou d'isolement peuvent être évitées ou réduites, diminuant ainsi considérablement le nombre de manipulations, de pipetages et le coût.

La présente Invention a donc pour objet l'utilisation d'un dispositif miniature de séparation et/ou d'isolement d'objets biologiques, comportant au moins une première électrode intégrée au dispositif et au moins une deuxième électrode intégrée ou externe au dispositif, constitué par une structure pourvue d'une matrice de microcuvettes réactionnelles, chaque microcuvette comportant un fond constituant une zone de réception, caractérisé en ce que ledit fond est dépourvu de trou et que la surface maximale dudit fond de chaque microcuvette est définie de manière à isoler un objet biologique unique, ladite structure étant reliée à un circuit d'alimentation pour créer une différence de potentiel entre ladite première électrode et ladite deuxième électrode, pour des applications liées à la biologie moléculaire, notamment pour la fabrication de puces à acides nucléiques (puces à ADN), la fabrication de puces à protéines, le tri de banques génomiques, l'analyse de banques de transcrits, la mesure d'une variation de l'activité d'une protéine fonctionnelle, la réalisation d'antivirogrammes et pour le criblage de protéines ou le criblage pharmaceutique.

Grâce au dispositif utilisé conformément à l'Invention, dans lequel chaque microréservoir possède au moins une microcuvette, il est désormais possible de ne fixer qu'un seul objet biologique par microcuvette, étant donné que la surface d'accroche de l'objet biologique à fixer recouvre la zone de réception, chaque microcuvette ne contenant donc qu'un seul type d'objet biologique sélectionné.

Par conséquent, selon l'Invention, la zone d'accroche de l'objet biologique à fixer ou la zone de recouvrement par l'objet biologique du fond de la microcuvette a soit une surface sensiblement identique à la surface de la zone de réception, soit une surface supérieure à la surface de la zone de réception.

Selon l'Invention, la surface maximale du fond de chaque microcuvette est de préférence inférieure ou égale à deux fois la plus petite surface de l'objet biologique à isoler. Dans une forme de mise en oeuvre préférée de l'Invention, la surface dudit fond est inférieure ou égale à la plus petite surface de l'objet biologique à isoler.

Plus particulièrement, cette surface est généralement comprise entre 1 µm² et 400 µm² et plus préférentiellement entre 1 et 50 µm².

Les objets biologiques sélectionnés et fixés peuvent ensuite être traités collectivement de façon à mettre en oeuvre des applications liées à la biologie moléculaire comprenant notamment l'élimination des objets biologiques non fixés, la séparation et/ou l'isolement d'objets biologiques recombinants, la libération du matériel génétique des objets biologiques par exemple par lyse chimique ou électrique, une étape d'amplification d'une partie ou du matériel génétique qu'ils contiennent.

Dans le cas de l'utilisation du dispositif conforme à l'Invention pour séparer et/ou isoler des objets biologiques recombinants, on utilise, avant l'étape de fixation des objets biologiques, une ou la combinaison de ces deux formes de mise en oeuvre :
- un vecteur de clonage pour transformer les objets biologiques portant un gène qui code pour une protéine spécifique qui sera présentée en surface de l'objet biologique (par fusion avec le début du gène Ipp par exemple) de telle sorte que la fixation des objets biologiques se fera par une interaction entre cette protéine et un réactif tel que défini dans le dispositif conforme à l'Invention (petite molécule ou anticorps spécifique). Les objets biologiques non transformés ne seront alors pas fixés sur le dispositif miniature conforme à l'Invention ;
- le clonage d'un fragment d'ADN dans le vecteur de clonage, ledit fragment empêchant l'expression d'un gène codant pour une protéine toxique pour l'objet biologique recombinant.

Au sens de la présente Invention, on entend par applications liées à la biologie moléculaire, des applications permettant d'obtenir des ARN, des ADN ou des protéines sur les dispositif conforme à l'Invention, notamment l'électroporation chimique ou électrique, la microinjection par microcapillaire, la lyse d'objets biologiques, l'amplification d'acides nucléiques utilisant par exemple des réactions de type PCR (polymerase chain reaction) ou NASBA (nucleic acid sequence-based amplification), la réplication d'acides nucléiques par cercle roulant, la transcription et la traduction de gènes, etc... Ces applications peuvent également correspondre à des réactions de transcription d'ADN en ARN messagers et à des réactions de traduction d'ARN messagers en protéines. Elles peuvent également concerner d'autres concepts tels que l'expression de protéines recombinantes, le tri d'une banque génomique ou d'une culture cellulaire en fonction de leur composition en acides nucléiques ou protéique, le criblage (de protéines recombinantes, enzymatique ou pharmaceutique), notamment pour la recherche de ligands protéiques ou nucléotidiques d'une cible donnée, le diagnostic, des applications de type "differencial display", puce à acides nucléiques, puce à protéines, l'analyse de banques de transcrits, le double hybride, la mesure d'une variation de l'activité d'une protéine fonctionnelle, etc...

En particulier, ce dispositif peut être utilisé pour le criblage d'enzymes.

Le dispositif utilisé conformément à l'Invention permet la fixation d'un seul objet biologique d'intérêt sur la zone de réception qui constitue en fait une zone piège.

Selon l'Invention, la matrice de microcuvettes réactionnelles du dispositif peut être surmontée au moins en partie d'une ou plusieurs couches de matériaux isolants et/ou d'une grille en plastique biocompatible rapportée, de façon à former une matrice de microréservoirs. Les matériaux isolants peuvent par exemple être choisis parmi les polymères isolants tels que les polyimides et les résines telles que par exemple les résines SU-8.

Selon l'Invention, un objet biologique est caractérisé par son contenant et son contenu. Le contenant correspond à tout élément permettant de compartimenter le contenu. Le contenant peut par exemple être la paroi d'une cellule bactérienne, l'enveloppe d'un virus, la membrane d'une cellule, une double couche lipidique, des micelles, une bicouche phospholipidique traversée par des protéines intrinsèques, etc. le contenu correspond au matériel biologique isolé dans un compartiment qu'est le contenant. Le contenu peut par exemple correspondre à des acides nucléiques, des protéines, des ribosomes, des vésicules membranaires ou à un mélange complexe de ceux-ci.

A titre d'exemple d'objet biologique, on peut citer toute cellule, saine ou non, qu'elle soit procaryote ou eucaryote, les virus, les liposomes, etc...

A titre d'exemple de cellule, on peut citer les bactéries, les levures, les champignons, les microalgues et également des cellules d'origine végétale, animales et humaines.

A titre d'exemple de virus, on peut citer le virus HIV, les bactériophages, etc...

La deuxième électrode interne ou externe de ce dispositif permet également la fixation ultérieure d'un ou plusieurs éléments dérivés de l'objet biologique d'intérêt préalablement fixé, ces dérivés incluant les produits issus de la lyse des objets biologiques, et/ou de leur traitement par une méthode d'amplification telle que par exemple la PCR localisée ou tout autre traitement biologique, chimique ou électrique. Lorsque ces dérivés correspondent à des acides nucléiques, on parle de puces à acides nucléiques ou à ADN. Lorsque ces dérivés correspondent à des protéines, on parle de puce à protéines.

La taille des microréservoirs est définie de manière à traiter, dans un volume minimal, l'objet biologique unique isolé. Ces microréservoirs ont généralement une largeur et/ou une longueur comprise entre 5 et 500 µm et préférentiellement entre 5 et 100 µm.

Selon une forme de réalisation particulière de l'Invention, le dispositif miniature utilisé peut comporter une alternance de couches conductrices (électrodes) et de couches de matériaux isolants.

Selon une forme de réalisation de l'Invention, une face de la première électrode intégrée au dispositif peut constituer le fond des microcuvettes.

Selon une autre forme de réalisation de l'Invention, le fond des microcuvettes du dispositif est constitué par une couche en verre, en plastique ou en silicium.

Lorsque le dispositif utilisé conformément à l'Invention comporte une deuxième électrode intégrée, celle-ci est déposée sur une première couche de matériau isolant et est située dans un plan espacé du fond des microcuvettes.

Lorsque le dispositif utilisé conformément à l'Invention comporte une deuxième électrode externe, celle-ci peut être solidaire d'un capot ou d'un couvercle, de préférence constitué par une ou plusieurs couches de matériau isolant.

Par conséquent une des couches de matériaux isolants peut ne pas faire partie intégrante du dispositif conforme à l'Invention mais se présenter sous la forme d'une pièce rapportée amovible (cache, capot, couvercle) venant recouvrir au moins en partie ledit dispositif et contenant éventuellement au moins une électrode.

Le dispositif utilisé conformément à l'Invention peut aussi comporter au moins une troisième électrode intégrée au dispositif, une deuxième couche de matériau isolant étant interposée entre la deuxième et la troisième électrode. Dans ce cas, et selon une variante de l'Invention, le dispositif peut comporter plusieurs deuxièmes et/ou troisièmes électrodes isolées entre elles.

Dans les dispositifs conformes à l'Invention, au moins un bord d'une des deuxièmes et/ou troisièmes électrodes et/ou d'une des premières et/ou deuxièmes couches de matériaux isolant peut constituer au moins une partie d'un bord d'un microréservoir.

Selon l'Invention, les premières, deuxièmes et troisièmes électrodes, ainsi que l'électrode externe sont constituées par au moins une couche métallique par exemple en chrome, en or ou en platine.

Ces couches métalliques ont en général une épaisseur comprise entre 0,1 et 10 µm.

Selon une forme de réalisation de l'Invention, un réactif apte à fixer l'objet biologique à isoler est fixé sur au moins une partie de la zone de réception des microcuvettes réactionnelles.

La nature du réactif utilisé pour la fixation des objets biologiques peut varier en fonction de leur nature et de la nature du fond des microcuvettes.

En effet, lorsque le fond des microcuvettes est constitué par une électrode telle que décrite ci-dessus, le réactif utilisé est de préférence choisi parmi les copolymères conducteurs, tels que par exemple les polypyrroles, sur lesquels sont fixés des protéines, des peptides ou toutes molécules spécifiques du type d'objet biologique à fixer telles que par exemple des anticorps, des récepteurs, des glycoprotéines, des lectines, des molécules d'adhésion cellulaire ("Cell Adhesion Molecules" : CAM), la laminine, la fibronectine, les intégrines, les sucres, etc...

Au sein d'un même dispositif, les réactifs des microcuvettes peuvent être identiques ou différents.

Les copolymères conducteurs sont par exemple décrits dans la demande internationale WO 94/22889.

Les poly pyrroles sont particulièrement préférés selon l'Invention.

Parmi les peptides pouvant être fixés sur les monomères du copolymère conducteur, on peut notamment citer les peptides de liaison spécifiques aux objets biologiques à isoler, tels que le fragment du complément C₃b et tels que les récepteurs membranaires de surface de l'objet biologique comme des peptides contenant la séquence Arginine-glycine-aspartate (encore appelée RGD).

Les molécules spécifiques fixées sur les monomères du copolymère conducteur peuvent notamment être choisies parmi la protéine A et la protéine G.

La fixation des molécules spécifiques sur les monomères du copolymère conducteur et en particulier sur les monomères pyrroles peut être effectuée suivant différentes techniques :
- soit les molécules spécifiques sont fixées directement sur les monomères d'un polymère conducteur lesdits monomères étant porteurs de fonctions
- NHS ou aldéhyde capables de réagir avec les fonctions amines primaires de la molécule spécifique utilisée,
- soit les molécules spécifiques sont fixées indirectement sur les monomères d'un polymère conducteur porteur de la fonction biotine, au moyen d'un empilement chimique successif streptavidine-biotine-molécule spécifique. Pour réaliser cet empilement chimique, le dispositif utilisé conformément à l'Invention est alors traité collectivement de façon à réaliser la copolymérisation des monomères du polymère conducteur porteur de la fonction biotine, puis à traiter ledit dispositif par de la streptavidine puis avec une molécule spécifique liée à la biotine, pour obtenir des copolymères pyrrole-biotine-streptavidine-biotine-molécule spécifique.

Dans une première forme de mise en oeuvre, le réactif utilisé pour fixer l'objet biologique peut être spécifique de celui-ci pour permettre une interaction directe : réactif de la microcuvette-objet biologique.

Dans une deuxième forme de mise en oeuvre, le réactif utilisé n'est pas spécifique de l'objet biologique. Celui-ci devra donc être préalablement fonctionnalisé par exemple par des anticorps spécifiques pouvant réagir avec les réactifs utilisés. Le réactif fixé uniquement sur la zone piège par l'intermédiaire du polymère conducteur, tel que par exemple la protéine A ou la protéine G, va reconnaître le fragment Fc des anticorps fixés préalablement sur les objets biologiques à immobiliser.

Lorsque le fond des microcuvettes est constitué par une couche en verre, en plastique ou en silicium, le réactif utilisé est de préférence :
- un polymère non spécifique du type d'objet biologique à fixer tel que par exemple de la poly-L-lysine ; ledit polymère étant déposé localement sur les zones de réception (technique "lift-off": dépôt d'une résine photo-imageable, insolation localisée puis dépôt du polymère sur la résine, puis déblocage de la résine),
- une protéine ou un peptide ; dans ce cas les protéines et les peptides sont fixés à ladite couche en verre, en plastique ou en silicium recouverte d'une couche de silane modifié par des fonctions -NHS ou aldéhyde sur lesquelles est fixé ledit réactif ; les protéines et les peptides utilisés dans ce cas étant de même nature que ceux décrits ci-dessus.

Selon une deuxième forme de réalisation de l'Invention, la zone de réception des microcuvettes réactionnelles ne comporte pas de réactif apte à fixer l'objet biologique que l'on désire isoler.

Dans ce cas, la fixation des objets biologiques est directement réalisée par l'intermédiaire d'un champ électrique.

Cette forme de réalisation est particulièrement avantageuse car elle évite la fonctionnalisation préalable des dispositifs conformes à l'Invention par un réactif apte à fixer l'objet biologique à isoler. Cette forme de réalisation est tout particulièrement bien adaptée à l'isolement et à la fixation de bactéries.

La présence d'au moins une première électrode intégrée au dispositif et d'au moins une deuxième électrode intégrée ou externe au dispositif utilisé conformément à l'Invention permet d'effectuer un traitement identique dans toutes les microcuvettes, tel que par exemple l'application d'un champ électrique de lyse des objets biologiques, un champ électrique de copolymérisation ou un champ électrique de perméabilisation membranaire.

En particulier, ces électrodes peuvent permettre la fixation spécifique d'objets biologiques tels que par exemple des micro-organismes, puis une fois les micro-organismes fixés dans les microcuvettes, la lyse ou la perméabilisation de ceux-ci, la fixation par copolymérisation électrique des sondes nucléiques issues d'une amplification génomique effectuée directement dans chacun des microréservoirs, de façon à obtenir des microréservoirs porteurs de sondes nucléiques en grande quantité.

La deuxième électrode présente dans tous les dispositifs utilisés conformément à l'Invention peut être soit préfonctionnalisée par exemple par des anticorps spécifiques pour extraire de chaque objet biologique une protéine spécifique, soit plus généralement être utilisée pour fixer par électrochimie un produit issu de l'objet biologique à la suite par exemple d'une réaction d'amplification ou de transcription.

Les différentes applications pour lesquelles sont utilisés les dispositifs miniatures décrits précédemment peuvent nécessiter de faire des répliques du matériel génétique des objets biologiques isolés.

Ces répliques peuvent être réalisées de différentes manières :
- soit les différents objets biologiques isolés sur un premier dispositif (objets biologiques pères) sont mis en culture ; on obtient alors une population homogène de chaque objet biologique au niveau de chaque microréservoir. Les objets biologiques issus de la multiplication cellulaire, encore appelés objets biologiques fils, peuvent être récupérés au niveau d'un second dispositif, par exemple par pipetage, pour constituer la copie conforme du premier dispositif ;
- soit le matériel génétique de chaque objet biologique père est amplifié par exemple par PCR, en utilisant des amorces portant un monomère d'un polymère conducteur. Les produits d'amplification pourront ainsi facilement être récupérés grâce à un champ électrique et disposé au sein d'un nouveau dispositif puis fixés sur une électrode dudit nouveau dispositif ;
- soit le matériel génétique de chaque objet biologique est transcrit en ARNs messager sur un premier dispositif (puce mère), puis les ARNs messagers ainsi obtenus sont transférés par pipetage collectif ou par application d'un champ électrique dans un second dispositif miniature neuf (puce fille) et fixés sur une électrode dudit nouveau dispositif

Les dispositifs miniatures décrits ci-dessus peuvent également être utilisés :
- pour exprimer à l'intérieur ou à l'extérieur des objets biologiques une partie de leur ou leur matériel génétique ; cette expression comprend notamment les étapes de transcription et de traduction :

- pour détecter au moins une propriété de la ou des protéines isolées sur le dispositif miniature conforme à l'Invention par révélation de ladite propriété ;
- dans le cadre des procédés définis dans les demandes internationales WO 00/09747, WO 00/34512, WO 00/34514 et WO 00/34513 ;
- pour l'analyse de transcrits comprenant une étape de détection d'un gène ayant subi une différence d'expression ;
- pour la mesure d'une variation de l'activité d'une protéine fonctionnelle caractérisée en ce que l'on teste l'effet de différentes molécules sur l'activité de ladite protéine fonctionnelle ;
- pour le criblage protéique ou pharmaceutique notamment pour la recherche d'enzymes ou de ligands protéiques ou nucléotidiques d'une cible donnée.

La révélation des protéines produites au cours de ce procédé peut se faire de différentes manières :
- le substrat de la protéine exprimée que l'on souhaite révéler spécifiquement est introduit dans les objets biologiques isolés par un choc électrique ;
- les protéines produites exprimées au cours de ce procédé sont évacuées des objets biologiques isolés, par un choc électrique au niveau des microréservoirs pour pouvoir être révélées spécifiquement en présence de leur substrat qui pourra par exemple être fixé au niveau d'un microréservoir ;
- les objets biologiques sont lysés et les protéines exprimées sont révélées spécifiquement en présence de leur substrat qui pourra par exemple être fixé au niveau d'un microréservoir.

Cette forme de mise en oeuvre du procédé est avantageuse car elle permet de séparer et de caractériser les protéines fonctionnelles codées par les acides nucléiques d'un échantillon brut d'objets biologiques.

Uniquement les protéines recombinantes peuvent être fixées sur les puces à protéines en utilisant, avant l'étape de fixation des objets biologiques, un vecteur de clonage pour transformer les objets biologiques permettant le marquage de la protéine recombinante par un épitope universel comme par exemple le marquage par plusieurs acides aminés histidine.

Les dispositifs miniatures utilisés conformément à l'Invention sont de préférence équipés d'un moyen de fermeture, tel que par exemple un capot ou un film transparent, permettant d'obturer individuellement ou collectivement tous les microréservoirs.

L'utilisation de dispositifs comportant une première électrode intégrée au dispositif et une deuxième électrode externe à celui-ci est particulièrement adaptée à la perméabilisation, à la libération du matériel génétique ou à la lyse des objets biologiques fixés, à la copolymérisation des acides nucléiques ou des protéines issus des objets biologiques ou des différents procédés de biologie moléculaire.

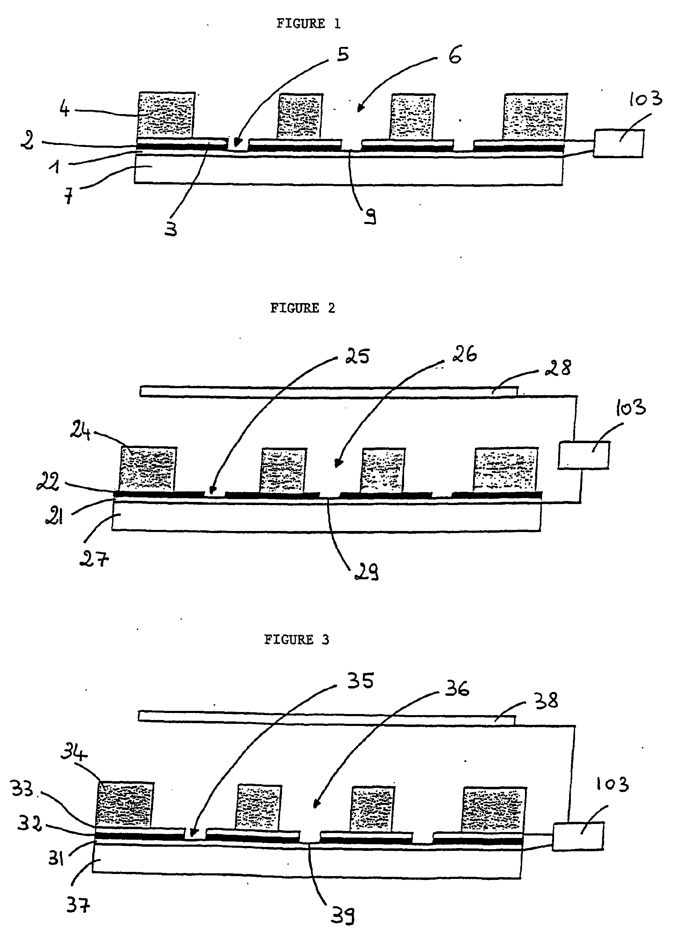

D'autres caractéristiques des dispositifs miniatures utilisés conformément à l'Invention apparaissent sur les figures 1 à 6 annexées dans lesquelles :
- la figure 1 représente un dispositif miniature selon l'Invention, équipé d'un support 7 et d'un circuit d'alimentation électrique 103, dans lequel le fond de chaque microcuvette 5 est constitué par une première électrode 1 formant une zone de réception 9 sur laquelle est éventuellement fixé un réactif, la première électrode 1 étant surmontée par une première couche de matériau isolant 2 sur laquelle repose une deuxième électrode 3 surmontée d'une deuxième couche de matériau isolant 4 formant des microréservoirs 6,
- la figure 2 représente un dispositif miniature selon l'Invention, équipé d'un support 27 et d'un circuit d'alimentation électrique 103, dans lequel le fond de chaque microcuvette 25 est constitué par une première électrode 21 formant une zone de réception 29 sur laquelle est éventuellement fixé un réactif, la première électrode 21 étant surmontée par une première couche de matériau isolant 22 sur laquelle repose une deuxième couche de matériau isolant 24 formant des microréservoirs 26, ce dispositif étant équipé d'une deuxième électrode externe 28,
- la figure 3 représente un dispositif miniature selon l'Invention, équipé d'un support 37 et d'un circuit d'alimentation électrique 103, dans lequel le fond de chaque microcuvette 35 est constitué par une première électrode 31 formant une zone de réception 39 sur laquelle est éventuellement fixé un réactif, la première électrode 31 étant surmontée par une première couche de matériau isolant 32 sur laquelle repose une deuxième électrode 33 surmontée d'une deuxième couche de matériau isolant 34 formant des microréservoirs 36, ce dispositif étant équipé d'une électrode externe 38,

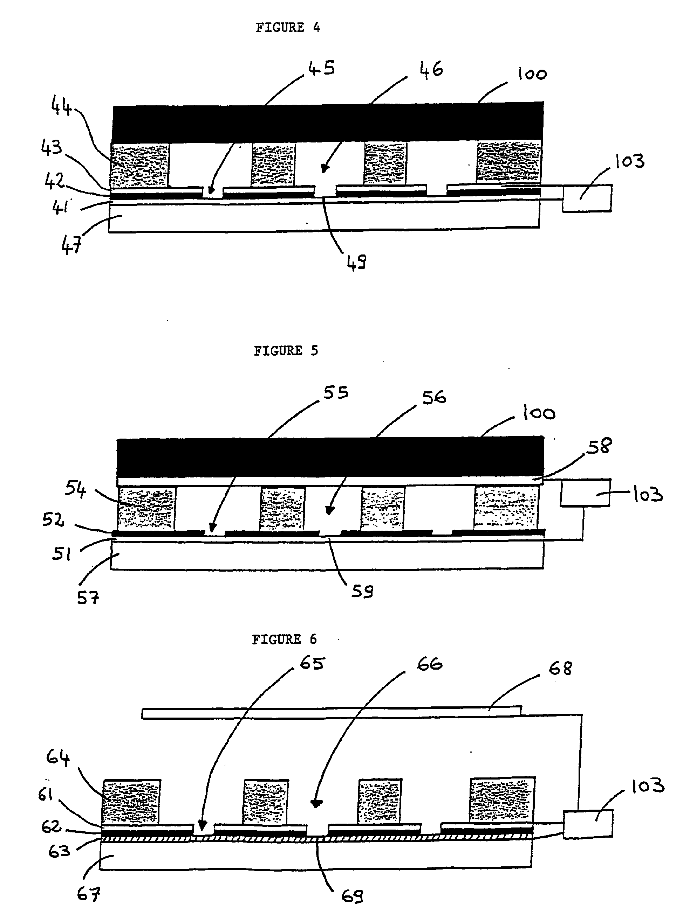

- la figure 4 représente un dispositif miniature selon l'Invention identique à celui représenté sur la figure 1 sauf qu'il comporte en outre un moyen de fermeture amovible 100 permettant de fermer chacun des microréservoirs 46,
- la figure 5 représente un dispositif miniature selon l'Invention identique à celui représenté sur la figure 2 sauf qu'il comporte en outre un moyen de fermeture amovible 100 permettant de fermer chacun des microréservoirs 46 dans lequel est intégré une deuxième électrode externe 58,
- la figure 6 représente un dispositif miniature selon l'Invention, équipé d'un support 67 et d'un circuit d'alimentation électrique 103, dans lequel le fond de chaque microcuvette 65 est constituée par une couche de verre ou de silicium 63 formant une zone de réception 69 sur laquelle est éventuellement fixé un réactif, ladite couche de verre ou de silicium 63 étant surmontée par une première couche de matériau isolant 62 formant des microréservoirs 66 sur laquelle repose une première électrode 61 elle-même surmontée par une deuxième couche de matériau isolant 64, ce dispositif étant équipé d'une deuxième électrode externe 68.

Il est bien sur entendu que les dispositifs illustrés sur ces figures correspondent à des formes particulières de réalisation de l'Invention et n'en constituent en aucune façon une limitation.

Les procédés de fabrication de tels dispositifs sont connus et décrits par exemple dans la demande de brevet FR-A-2 781 886.

Selon l'Invention, les objets biologiques à isoler peuvent être choisis parmi les cellules saines ou non, comme par exemple des cellules infectées par des virus ou des cellules malignes. Ces objets biologiques peuvent donc correspondre à des cellules procaryotes ou eucaryotes, des virus, des liposomes ou des microalgues. Selon une forme de réalisation particulièrement préférée de l'Invention, les objets biologiques à isoler sont choisis parmi les bactéries et les levures, les bactéries étant préférentiellement issues d'une banque de séquences génomiques.

Selon une forme de réalisation particulière de l'Invention, ces dispositifs miniatures peuvent être utilisés pour l'obtention de puces à acides nucléiques ou à protéines ou bien pour la détection de protéines fonctionnelles. Ces puces à ADN ou à protéines constituent un autre objet de l'Invention.

L'Invention a donc également pour objet un procédé de séparation et/ou d'isolement d'objet biologique pour l'obtention de puces à acides nucléiques ou à protéines ou pour la détection de protéines fonctionnelles caractérisé en ce qu'il comprend au moins les étapes a) et b) suivantes :
a) la mise en contact d'au moins un dispositif miniature tel que défini précédemment avec une solution d'objets biologiques homogénéisée, en particulier avec une solution de culture de cellules biologiques, pour permettre la fixation desdits objets biologiques au fond des microcuvettes sur les zones de réception, à raison d'au plus un objet biologique par microcuvette,
b) l'élimination des objets biologiques non fixés de façon à obtenir un dispositif miniature sur lequel les objets biologiques à isoler sont immobilisés.

Selon une première forme de réalisation du procédé conforme à l'Invention, la fixation des objets biologiques est réalisée par l'intermédiaire d'un champ électrique. Dans ce cas, on utilise de préférence des dispositifs dans lesquels la première électrode intégrée au dispositif constitue le fond des microcuvettes.

Selon une deuxième forme de réalisation du procédé conforme à l'Invention, la fixation des objets biologiques est réalisée par l'intermédiaire d'un réactif fixé sur au moins une partie du fond des microcuvettes réactionnelles. Dans ce cas le fond des microcuvettes peut aussi bien être constitué par une première électrode ou par une couche en verre, en plastique ou en silicium.

Le procédé conforme à l'Invention peut comporter une étape préliminaire à l'étape a), consistant à transformer les objets biologiques par un vecteur de clonage portant un gène qui code pour une protéine spécifique qui sera présentée en surface des objets biologiques de telle sorte que la fixation des objets biologiques se fera par une interaction entre cette protéine et un réactif spécifique de ladite protéine et tel que défini précédemment et/ou par clonage d'un fragment d'ADN dans un vecteur, ledit fragment empêchant l'expression d'un gène codant pour une protéine toxique pour l'objet biologique recombinant.

Selon une forme de réalisation préférée de l'Invention, on utilise de un dispositif comportant des microréservoirs et ce procédé comporte une étape au cours de laquelle le matériel génétique des objets biologiques est libéré dans lesdits microréservoirs par toute technique connue de l'homme du métier (lyse, choc électrique, etc...).

Les dispositifs utilisés au cours de ce procédé comportent également de préférence une deuxième électrode interne ou externe adaptée à la perméabilisation des objets biologiques fixés.

Lorsque ce procédé est utilisé pour l'obtention de puces à acides nucléiques, il comporte alors une étape au cours de laquelle le matériel génétique des objets isolés est amplifié pour obtenir des séquences amplifiées. Cette étape d'amplification est en général suivie d'une étape de fixation par électropolymérisation des séquences amplifiées sur une deuxième électrode du dispositif utilisé.

Lorsque ce procédé est utilisé pour l'obtention de puces à protéines ou pour la détection de protéines fonctionnelles, il comprend de préférence, après l'étape b), une étape de transcription et de traduction du matériel génétique des objets biologiques isolés en protéines. Les protéines ainsi exprimées peuvent être fixées, comme décrit précédemment, sur une deuxième électrode du dispositif utilisé.

Ce procédé peut en particulier être utilisé pour l'obtention de puces à protéines recombinantes, et dans ce cas, avant l'étape a), le procédé comporte alors une étape de transformation des objets biologiques par un vecteur de clonage afin de permettre le marquage des protéines recombinantes par un épitope universel.

Lorsque ce procédé est utilisé pour la détection de protéines fonctionnelles, il comprend alors, après l'étape de traduction, une étape de révélation d'au moins une propriété des protéines isolées.

Une mesure de la variation de l'activité des protéines fonctionnelles ainsi isolée peut également être réalisée selon une méthode consistant à tester l'effet de différentes molécules sur l'activité desdites protéines fonctionnelles.

Selon une forme de réalisation particulière de l'Invention, le procédé de fabrication d'une puce à acides nucléiques (puce à ADN) est caractérisé en ce qu'il comprend les étapes suivantes :
a) la mise en contact d'au moins un dispositif miniature tel que défini précédemment avec une solution d'objets biologiques homogénéisée, en particulier avec une solution de culture de cellules biologiques, pour permettre la fixation desdits objets biologiques au fond des microcuvettes sur les zones de réception, à raison d'au plus un objet biologique par microcuvette,
b) l'élimination des objets biologiques non fixés de façon à obtenir un dispositif miniature sur lequel les objets biologiques à isoler sont immobilisés,
c) l'introduction dans les microréservoirs d'un mélange réactionnel commun contenant tous les réactifs nécessaires à une amplification génomique, ledit mélange réactionnel comprenant notamment au moins une amorce fonctionnalisée par des groupements pyrrole,
d) la lyse ou la perméabilisation des objets biologiques fixés au fond des microcuvettes de façon à mettre en contact le matériel génétique qu'ils contiennent avec les réactifs nécessaires à une amplification des séquences,
e) la fixation des séquences amplifiées sur une deuxième électrode pour obtenir une puce à acides nucléiques.

Afin d'obtenir ces puces à ADN, il est possible d'utiliser les dispositifs tels que ceux illustrés par les figures 1, 3 et 6. Dans ce cas on utilise de préférence des dispositifs comportant des microcuvettes dont le fond à une surface d'environ 1 µm², la culture cellulaire étant alors préférentiellement une banque bactérienne de séquences nucléotidiques.

Le procédé conforme à l'Invention ne nécessite ni micropipetage, ni positionnement délicat des objets à isoler.

L'avantage de ce procédé est de fabriquer de manière collective toutes les sondes de la puce, la nature des sondes et leur représentativité étant directement reliées à la diversité de la culture de départ.

Par exemple, les cellules de départ (bactéries, levures) peuvent être issues de la transformation de cellules hôtes par une banque de vecteurs recombinants portants des séquences nucléotidiques (séquence génomique, séquence d'ADNc, séquence différentielle, etc....). Les sondes sont fabriquées par amplification des séquences nucléotidiques contenues dans les vecteurs recombinants grâce à des amorces incluses dans le vecteur. La puce est alors utilisée comme une matrice d'étalement d'une banque de clones. La puce est aléatoire dans le sens où l'on ne connaît pas la nature exacte de chaque sonde dans chaque microcuvette. C'est le principe du clonage.

En effet, le clonage de bactéries ou de levures est habituellement réalisé par étalement des cellules sur un milieu nutritif solide. Les cellules sont ainsi individualisées spatialement. Après de nombreuses divisions (au-delà de 20 heures de culture au moins), la cellule individualisée a formé une colonie de cellules toutes identiques. La multiplication des cellules a ainsi induit indirectement l'amplification de la séquence présente dans le vecteur recombinant.

La difficulté de ce procédé de clonage classique est d'obtenir un étalement régulier de colonies bien indépendantes, au maximum 20-30 bactéries/cm² pour pouvoir ensuite isoler la colonie intéressante. Il faut ensuite remettre en culture cette colonie intéressante, ce qui nécessite encore quelques heures de culture supplémentaires.

Par contre, selon le procédé conforme à l'Invention, l'équivalent de 5000 clones/cm² est réalisable de façon rapide, avec un étalement ordonné. L'amplification peut se réaliser en 1 heure et le greffage par polypyrrole est instantané.

Les difficultés de culture de certains microorganismes, de transfert sur membrane et de fixation d'ADN sont ainsi évitées.

Les puces à ADN conformes à l'Invention peuvent par exemple être utilisées pour le criblage à grande échelle d'une banque de gènes.

Elles permettent en particulier :
- de trouver la séquence complète d'un gène à partir d'un fragment connu qui sera marqué et hybridé sur la puce : le ou les microréservoirs marqués (positifs) contenant la séquence du gène recherché ;
- de trouver des gènes homologues par hybridation peu stringente (gènes homologues inter-espèces ou gènes d'une même famille), à partir de la séquence d'un gène de départ qui sera marquée et hybridée sur la puce : le ou les microréservoirs positifs contenant la séquence de gènes homologues ;
- de déterminer la représentativité d'un gène particulier dans la banque en utilisant le gène en question comme sonde et en divisant le nombre de microréservoirs positifs par le nombre total de microréservoirs afin d'évaluer la qualité de la banque et d'évaluer les critères de représentativité des gènes en fonction de leur quantité et de leur taille ;
- d'évaluer la qualité d'une banque normalisée (tous les gènes étant représentés un même nombre de fois), la qualité d'une banque de soustraction (pas d'hybridation croisée sur les mêmes microréservoirs).

Les puces à ADN conformes à l'Invention peuvent également être utilisées pour le criblage d'ADN en fonction d'une protéine cible. Dans ce cas les sondes fixées sur la puce sont un panel de promoteurs. On cherche la séquence sur laquelle se fixe une protéine cible trans-régulatrice donnée. Cette protéine est marquée au préalable et on la met en incubation dans chaque microréservoir de la puce. Les microréservoirs positifs contiennent la séquence du promoteur spécifique de la protéine.

Les dispositifs conformes à l'Invention, et en particulier les dispositifs illustrés par les figures 1 et 3 dans lesquels la deuxième électrode a été fonctionnalisée avec un anticorps spécifique contre un épitope universel (anticorps anti-HA, anti-myc ou anti-poly-hystidine) peuvent également être utilisés pour la fabrication de puces à protéines.

Ces puces à protéines constituent un autre objet de l'Invention.

L'Invention a donc également pour objet un procédé de fabrication d'une puce à protéines caractérisé en ce qu'il comprend les étapes suivantes :
a) la mise en contact d'au moins un dispositif miniature tel que défini précédemment avec une solution de culture de cellules biologiques transformées par un vecteur recombinant portant un gène permettant le tri ultérieur des clones recombinants, ledit vecteur permettant en outre le marquage de la protéine recombinante par un épitope universel (de type poly-histidine par exemple), pour permettre la fixation desdites cellules au fond des microcuvettes sur les zones de réception, à raison d'au plus une cellule par microcuvette,
b) l'élimination des cellules non fixées de façon à obtenir un dispositif miniature sur lequel les cellules à isoler sont immobilisées,
c) l'introduction dans les microréservoirs des réactifs induisant la transcription et la traduction du matériel génétique de façon à permettre la synthèse des protéines correspondantes,
d) la libération des protéines recombinantes exprimées par exemple par lyse des objets biologiques fixés au fond des microcuvettes,
e) la fixation des protéines portant l'épitope universel au niveau d'une deuxième électrode interne au dispositif, celle-ci ayant préalablement été fonctionnalisée par exemple avec des anticorps antiépitope universel,
f) l'élimination éventuelle par lavage des autres constituants des objets biologiques, pour obtenir une puce à protéines.

Seules les protéines recombinantes sont retenues au niveau de cette puce.

La puce à protéines aléatoire ainsi obtenue peut être utilisée par exemple pour la réalisation de tests biochimiques ou de reconnaissance protéine/protéine avec une protéine cible marquée pour laquelle on veut identifier dans la banque les partenaires protéiques avec qui elle peut interagir, comme par exemple le double hybride ou encore de tests de reconnaissance protéine/ADN protéine/sucre, etc....

La fabrication des puces à protéines peut également être réalisée sans tri préalable de la banque.

Selon une forme de réalisation particulière, et lorsque l'on utilise par exemple les dispositifs illustrés par les figures 1 et 3, tous les réactifs peuvent être fixés par l'intermédiaire de groupements pyrroles. On effectue alors la synthèse de monomères pyrroles porteurs de fonctions -NHS que l'on couple par des liaisons NHS-NH₂ à différents anticorps, par exemple à un anticorps dirigé contre l'épitope universel, un autre anticorps étant spécifique de la cellule à immobiliser. On obtient ainsi par exemple un monomère pyrrole-anticorps 1 (dirigés contre la cellule à immobiliser) et un monomère pyrrole-anticorps 2 (dirigé contre l'épitope universel). Le dispositif conforme à l'Invention est alors immergé dans une solution contenant un mélange pyrrole/pyrrole-anticorps 1 et la première électrode est activée afin de fonctionnaliser le fond des microcuvettes.

Après rinçage, le dispositif conforme à l'Invention est ensuite immergé dans une solution contenant un mélange pyrrole/pyrrole-anticorps 2 et la deuxième électrode est activée afin de la fonctionnaliser.

Le dispositif ainsi fonctionnalisé est ensuite trempé dans une solution de bactéries. Par reconnaissance et encombrement stérique, une bactérie unique est alors fixée au fond de chacune des microcuvettes, sur la première électrode.

La traduction des protéines est alors effectuée intra-bactérie comme décrit ci-dessus, puis les bactéries sont lysées de façon à libérer les protéines épitope universel synthétisées qui, si elles sont reconnues par l'anticorps anticorps 2, seront fixées sur la deuxième électrode.

Selon une autre forme de réalisation particulière de ce procédé, et lorsque l'on utilise un dispositif comportant des microcuvettes dont le fond est par exemple constitué par une couche de verre, tel que celui représenté par la figure 6, il est possible de fixer la bactérie par un polymère non spécifique ou par silanisation NHS et fixation d'un anticorps 1 spécifique du type de cellule à immobiliser, puis on procède comme précédemment décrit.

Selon une variante de ce procédé, la fabrication de puces à protéines peut être réalisée en effectuant les étapes suivantes consistant en :
a) la mise en contact d'au moins un dispositif miniature tel que défini précédemment et dans lequel le fond des microcuvettes est constitué par une couche de verre, avec une émulsion dont chaque micelle contient un gène et le milieu réactionnel nécessaire pour effectuer l'expression du gène *in vitro* de façon compartimentée, pour permettre la fixation desdits micelles au fond des microcuvettes sur les zones de réception, à raison d'au plus un micelle par microcuvette,
b) l'élimination éventuelle des micelles non fixés de façon à obtenir un dispositif miniature sur lequel les micelles à isoler sont immobilisés,
d) la lyse des micelles fixés au fond des microcuvettes de façon à libérer les protéines exprimées,
e) la fixation des protéines au niveau d'une deuxième électrode interne au dispositif et fonctionnalisée,
f) l'élimination éventuelle par lavage des autres constituants des micelles pour obtenir une puce à protéines.

La préparation de cette émulsion peut être effectuée de manière connue et telle que décrite par exemple par Tawfik et Griffiths, Nature Biotechnology, 1998, 16, 652-656.

Une émulsion peut également être réalisée en présence d'une solution d'ADN de manière à compartimenter des fragments d'ADN dans des micelles.

Chaque micelle est ensuite isolé grâce à un dispositif miniature tel que décrit précédemment puis lysé de manière à libérer, au niveau de chaque microréservoir, le ou les fragments d'ADN qui pourront ensuite être localement transcrits puis traduits de manière à étudier la fonction des protéines ainsi obtenues. Cette forme de mise en oeuvre du dispositif de l'Invention permet de séparer et de caractériser des protéines fonctionnelles au niveau d'un dispositif miniature tel que décrit précédemment.

Selon encore une autre variante de l'Invention, les dispositifs miniatures conformes à l'Invention peuvent être utilisés pour fabriquer une puce à protéines aléatoire tout en gardant l'information de la séquence ADN codant pour la protéine qui aura été synthétisée comme précédemment, sous la forme d'une puce à ADN aléatoire.

Pour ce faire, les cellules de la banque sont immobilisées comme décrit ci-dessus au fond des microcuvettes d'une première puce A, sur laquelle on effectue quelques divisions cellulaires. Une copie de la première puce A est réalisée dans une deuxième puce B, par transfert de cellules de manière dirigée des microréservoirs de la puce A vers les microréservoirs correspondant de la puce B, les microréservoirs de la puce A étant éventuellement mis en vis-à-vis avec les microréservoirs de la puce B, sous agitation.

La puce A subit ensuite le traitement décrit ci-dessus pour la fabrication d'une puce à ADN aléatoire, tandis que la puce B subit le traitement décrit ci-dessus pour obtenir une puce à protéine aléatoire correspondante. Après identification des microcuvettes positives (soit par un test protéique sur la puce B, soit par un test ADN sur la puce A, on obtient alors directement la séquence de l'ADN sur la puce A ou celle de la protéine correspondante sur la puce B.

L'Invention a aussi pour objet un procédé de criblage de protéines, recombinantes ou non, caractérisé en ce qu'il comprend les étapes suivantes :
a) la mise en contact d'au moins un dispositif miniature (puce mère) tel que défini précédemment avec une solution d'objets biologiques homogénéisée, en particulier avec une solution de culture de micro-organismes, pour permettre la fixation desdits objets biologiques au fond des microcuvettes sur les zones de réception, à raison d'au plus un objet biologique par microcuvette,
b) l'élimination éventuelle des objets biologiques non fixés de façon à obtenir un dispositif miniature sur lequel les objets biologiques à isoler sont immobilisés,
c) l'introduction dans les microréservoirs d'un mélange réactionnel commun contenant tous les réactifs nécessaires à une amplification génomique, ledit mélange réactionnel comprenant notamment au moins une amorce fonctionnalisée par des groupements pyrrole,
d) la lyse ou la perméabilisation des objets biologiques fixés au fond des microcuvettes de façon à mettre en contact le matériel génétique qu'ils contiennent avec les réactifs nécessaires à une amplification et permettre ainsi ladite amplification,
e) l'introduction dans chaque microréservoir d'un mélange réactionnel contenant au moins une ARN polymérase permettant la transcription des séquences amplifiées en ARN messagers (transcrits),
f) l'introduction dans chaque microréservoir d'un mélange réactionnel permettant la traduction des transcrits obtenus à l'étape (e) en protéines correspondantes,
g) le test d'au moins une propriété des protéines obtenues à l'étape (f) par l'introduction dans chaque microréservoir d'un substrat approprié, et éventuellement
h) la lyse des objets biologiques si celle-ci n'a pas été effectuée lors de l'étape (d), puis le séquençage des gènes contenus dans les microréservoirs dans lesquelles une réaction positive protéine/substrat a eu lieu.

Selon ce procédé de criblage, les étapes (c) et (d) peuvent éventuellement être réalisées de façon simultanée.

Lorsque les objets biologiques ont été lysés à l'étape (d), alors le procédé conforme à l'Invention peut comporter une étape supplémentaire consistant à fixer les séquences amplifiées sur une électrode par électropolymérisation, de manière collective. Dans ce cas, une étape de lavage des microréservoirs peut ensuite être effectuée pour éliminer les restes des objets biologiques lysés et éventuellement les séquences amplifiées non fixées sur l'électrode.

Également, lorsque les objets biologiques ont été lysés à l'étape (d), le procédé de criblage conforme à l'Invention peut comporter une autre étape supplémentaire consistant à polariser une électrode pour attirer les ARN messagers synthétisés à l'étape (e) au fond des microréservoirs, cette étape de polarisation étant éventuellement suivie d'une étape de lavage des microréservoirs.

Le procédé de criblage conforme à l'Invention ne nécessite ni micropipetage, ni positionnement délicat des objets biologiques à isoler.

Ce procédé permet en effet le criblage de protéines sur une matrice ordonnée tout en ne nécessitant pas de pipetage individuel pour créer ladite matrice.

Il permet également de limiter le nombre des séquençages à effectuer, seuls les gènes contenus dans les microcuvettes ayant donné une réaction positive protéine/substrat étant séquencés.

Selon une forme de réalisation particulièrement préférée de ce procédé, le dispositif utilisé est muni d'un moyen de fermeture permettant de fermer les microréservoirs pendant le déroulement des différentes réactions (lyse ou perméabilisation des objets biologiques, amplification génomique, transcription des séquences amplifiées en ARN messagers, traduction des transcrits en protéines correspondantes, etc).

Les mélanges réactionnels permettant la transcription des séquences amplifiées en ARN messagers et la traduction des transcrits en protéines sont ceux classiquement utilisés pour réaliser ces opérations. Ils sont par exemple décrits en détail dans la demande internationale WO 00/09747.

Selon une forme de réalisation préférée de l'Invention, les étapes (e) et (f) sont réalisées simultanément.

Selon une variante avantageuse de ce procédé, et lorsque les objets biologiques ont été lysés à l'étape (d) et que les séquences amplifiées ont été fixées sur une électrode par électropolymérisation, alors les ARN messagers obtenus à l'issue de l'étape (e) peuvent être transférés, par exemple par pipetage collectif, dans un dispositif miniature vierge (puce fille) identique au précédent dans lequel auront lieu les étapes (f) et (g) telles que définies précédemment.

Ce transfert permet de préserver l'intégrité des séquences amplifiées dans les microréservoirs du premier dispositif. En effet, les réactifs mis en oeuvre au cours de l'étape (f) de traduction contiennent des DNases qui abîment l'ADN des séquences amplifiées interdisant ensuite l'exploitation des informations qu'elles contiennent.

Ce transfert peut notamment être effectué au moyen d'un outil négatif à pointes métalliques dans lesquelles on applique un champ électrique de façon à attirer les ARN messagers. Ces derniers seront alors déposés dans les microréservoirs de la puce fille par dépolarisation des pointes métalliques de cet outil. Les ARN messagers ainsi transférés sur la puce fille pourront alors être attirés sur une électrode de la puce fille au moyen de l'application d'un champ électrique.

La copie puce mère/puce fille peut également être réalisée suite à l'étape (b) et après quelques divisions cellulaires en présence d'un milieu de culture approprié, par la simple mise en vis-à-vis du dispositif miniature utilisé (puce mère) avec un autre dispositif miniature vierge du même type (puce fille). La puce mère et la puce fille subissent alors toutes deux les étapes (c) et (d) telles que décrites ci-dessus, tandis que seule la puce fille subit ensuite l'étape (e).

Lorsqu'une puce mère et une puce fille sont utilisées conformément à l'Invention, des dispositifs tels que celui représenté par la figure 2, c'est à dire comprenant une première électrode intégrée au dispositif et une deuxième électrode externe à celui-ci, peuvent être utilisés.

La puce fille reçoit alors collectivement le mélange réactionnel contenant les réactifs nécessaires à la traduction des ARN messagers en protéines correspondantes (acides aminés marqués) et des vésicules porteurs d'un substrat réactif, tels que par exemple des liposomes, pour tester l'activité des protéines synthétisées.

Une fois que la réaction de traduction des protéines a eu lieu, une première vérification de la synthèse des protéines est effectuée microréservoir par microréservoir, par exemple par mesure en fluorescence polarisée, afin de discréminer le rayonnement émis en fonction de la taille des molécules (protéines/acides aminés marqués).

Le substrat réactif pour tester l'activité des protéines synthétisées est alors libéré localement dans chaque microréservoir par éclatement des vésicules sous l'action d'une sonication. Ce substrat est ensuite modifié ou non par l'action des protéines synthétisées et la modification de ce substrat est détectée par tout moyen approprié.

Ce procédé permet donc de trouver quel microréservoir a généré la protéine responsable de la modification du substrat testé. Il suffit ensuite de séquencer la séquence amplifiée fixée dans le microréservoir (microréservoir positif) correspondant de la puce mère.

La puce mère subit alors un nouveau cycle d'amplification génomique en utilisant des amorces ne contenant pas de fonction pyrroles.

Le contenu des microréservoirs positifs peut ensuite être pipeté localement et distribué dans des tubes individuels où un séquençage classique pourra être réalisé.

Lorsque les dispositifs miniatures tels que définis précédemment sont utilisés pour effectuer l'analyse d'une banque de transcrits, ils permettent de déterminer quels sont les gènes qui subissent un différentiel d'expression en réponse à un stress tel que par exemple un choc thermique, une exposition à une drogue, une pathologie.

L'Invention a donc également pour objet un procédé d'analyse d'une banque de transcrits caractérisé par le fait qu'il comprend les étapes a) et b) décrites précédemment (fixation des objets biologiques sur un dispositif miniature et lavage des objets biologiques non fixés) ainsi qu'une étape de détection d'un gène ayant subi une différence d'expression.

Ce procédé est de préférence caractérisé par le fait que dans une première étape, on effectue les sous-étapes suivantes consistant :
- à isoler des objets biologiques A sur un premier dispositif miniature tel que défini précédemment (puce mère A),
- à laver les objets biologiques A non fixés de façon à obtenir un dispositif où sont immobilisés les objets biologiques A à isoler, puis
- à extraire les ARNs messagers des objets biologiques A (cellules A dites stressées), par exemple en effectuant une lyse des différents objets isolés;
- à soumettre le contenu cellulaire à un traitement à la DNase,
- à transférer les ARNs messagers extraits dans les microréservoirs d'un dispositif neuf (puce fille A), par exemple au moyen d'un outil négatif à pointes métalliques dans lequel on applique temporairement un champ électrique de façon à attirer puis à déposer les ARNs messagers,
- à attirer les ARNs messagers sur une électrode de la puce fille A au moyen d'un champ électrique,
- à synthétiser les ADNc correspondants,
- à marquer les ADNc ainsi synthétisés à l'aide d'un segment nucléotidique comportant au moins un promoteur de transcription *in vitro* et au moins un segment permettant leur fixation spécifique dans un microréservoir de la puce fille A.

Chaque brin d'ADNc marqué est ainsi fixé au niveau des microréservoirs de la puce fille A.

Ces mêmes opérations peuvent être effectuées à partir d'objets biologiques B (cellules identiques aux cellules A, mais non stressées). De la même façon les d'ADNc correspondants seront fixés au niveau des microréservoirs d'une puce fille B.

Dans une deuxième étape, la puce fille A est alors plongée dans une solution contenant les brins non marqués des ADNc de la cellule B. Si un brin non marqué s'hybride sur son brin complémentaire sur la puce fille A, il permet de reconstituer un promoteur de transcription double brin fonctionnel.

La réalisation d'une étape de transcription *in vitro* dans chaque microréservoir, en utilisant des nucléotides fluorescents, permet de mettre en évidence quel microréservoir contient un ADNc de la cellule B capable de s'hybrider sur un ADNc de la cellule A.

En parallèle la puce fille B est plongée dans une solution contenant les brins non marqués des ADNc de la cellule A et la même révélation est alors réalisée. On met ainsi en évidence quel microréservoir contient un ADNc de la cellule A capable de s'hybrider sur un ADNc de la cellule B.

Dans une troisième étape, les puces filles A et B sont traitées pour éliminer les ARNm produits, ainsi que les ADNc non marqués.

Les puces filles A et B sont ensuite plongées dans une solution contenant les brins non marqués des ADNc des cellules A et B respectivement, et une nouvelle révélation par transcription est réalisée.

Le signal obtenu dans chaque microréservoir est alors comparé au signal qui a été obtenu à l'étape précédente, et le matériel génétique présent dans les microréservoirs négatifs à l'issue de la deuxième étape et positifs à l'issue de la troisième étape est récupéré pour être caractérisé par exemple par la réalisation d'une transcription inverse afin de séquencer les gènes ayant subi un différentiel d'expression.

Les dispositifs miniatures tels que décrits précédemment peuvent également être utilisés pour la réalisation d'antivirogrammes.

Les antivirogrammes consistent à définir les inhibiteurs d'une enzyme virale indispensable au cycle de multiplication d'un virus, permettant ainsi l'identification de principes actifs virucides, dans une forme de mise en oeuvre correspondant à l'infection de cellules par une forme unique d'un virus.

L'Invention a donc également pour objet un procédé de réalisation d'un antivirogramme, caractérisé par le fait qu'il comprend les étapes suivantes consistant à :
- isoler des cellules potentiellement infectées ou infectées sur un dispositif miniature tel que défini précédemment,
- à lyser lesdites cellules afin de libérer le gène codant pour l'enzyme indispensable au cycle de multiplication du virus,
- à amplifier ledit gène par exemple par toute technique connue de l'homme du métier,
- à fixer les produits d'amplification au niveau d'une deuxième électrode ; des polymères de copolymérisation seront par exemple utilisés à cet effet,
- à éliminer éventuellement par lavage les autres constituants des cellules de manière à ne retenir que les produits d'amplification,
- à transcrire puis traduire en protéines lesdits produits d'amplification au niveau des microréservoirs,
- à révéler l'activité des protéines obtenues en présence d'inhibiteurs identiques ou non au niveau de chaque microréservoir de manière à mettre en évidence les inhibiteurs capables d'inhiber *in vitro* une enzyme virale indispensable à la multiplication d'un virus.

Ce type d'expérience permet également d'étudier la représentativité de l'enzyme dans le cas où plusieurs isoformes de cette dernière existeraient.

Les dispositifs miniatures tels que décrits précédemment peuvent également être utilisés selon une autre forme de mise en oeuvre d'un antivirogramme.

Dans ce cas, les cellules d'une banque transformées avec les gènes codant pour les différentes isoformes d'une enzyme virale indispensable au cycle de multiplication d'un virus sont isolées grâce à un dispositif miniature tel que décrit ci-dessus. Seules les cellules recombinantes sont isolées (soit par tri de la banque tel que décrit ci-avant, soit par sélection préalable sur milieu sélectif). Les cellules non retenues au niveau du dispositif sont éliminées par lavage. Les protéines codant pour l'enzyme virale peuvent ensuite être exprimées et étudiées comme préalablement pour donner un antivirogramme de l'enzyme virale étudiée.

Outre les dispositions qui précèdent, l'Invention comprend encore d'autres dispositions qui ressortiront de la description qui va suivre, qui se réfère à des exemples d'immobilisation de bactéries sur des dispositifs miniatures conformes à l'Invention, à un exemple décrivant le protocole de préparation d'une puce à ADN sur un dispositif conforme à l'Invention, ainsi qu'à un exemple d'expression d'un matériel génétique sur un dispositif miniature conforme à l'Invention.

### EXEMPLE 1 : ISOLEMENT ET FIXATION DE BACTÉRIES SUR UN DISPOSITIF MINIATURE PAR L'INTERMÉDIAIRE DE PROTÉINES A

On prépare une solution de protéine A à 0,1 mg/ml dans du tampon phosphate (PBS).

A l'aide d'une pipette, on dépose ensuite une goutte de cette solution de protéine A sur un dispositif miniature conforme à l'Invention et tel que décrit à la figure 1 annexée, de telle sorte que ladite goutte recouvre l'ensemble des microcuvettes.

Sur ce dispositif, chaque microréservoir présente un diamètre de 230 µm et une profondeur de 40 µm ; la surface du fond de chaque microcuvette étant de 40 µm².

On applique ensuite pendant 10 secondes, un champ électrique entre les deux électrodes de la puce : potentiel de + 2,9 V sur l'électrode où l'on désire fixer la protéine A, l'autre électrode étant mise à la masse.

Lorsque la fixation est réalisée, le dispositif est alors rincé avec une solution de PBS.

Par ailleurs, on prépare une solution de PBS renfermant un complexe bactéries/anticorps.

Pour ce faire, on prépare d'abord une solution d'*E. Coli* DH5α dans du PBS (10⁹ bactéries/ml), ainsi qu'une solution de l'anticorps correspondant Anti E. Coli (Dako) à 0,5 mg/ml.

Ces deux solutions sont ensuite mélangées (V/V) et laissées sous agitation à température ambiante pendant 1 heure et trente minutes afin de former le complexe bactéries/anticorps. Le complexe bactéries/anticorps est ensuite concentré par centrifugation, les anticorps en excès étant éliminés en retirant le surnageant. L'opération est répétée trois fois, après remise en solution du complexe bactéries/anticorps dans du PBS.

A l'aide d'une pipette, on dépose ensuite une goutte de la solution contenant le complexe bactéries/anticorps sur le dispositif miniature fonctionnalisé par la protéine A, de telle sorte que ladite goutte recouvre l'ensemble des microcuvettes.

Le dispositif miniature est ensuite laissé à incuber pendant 1 heure et 30 minutes à température ambiante, afin de permettre l'immobilisation du complexe bactérie/anticorps au fond des microcuvettes.

À l'issue de l'incubation, le dispositif est alors rincé abondamment au PBS afin d'éliminer les complexes bactéries/anticorps n'ayant pas réagi avec la protéine A.

On obtient un dispositif sur lequel sont immobilisées des bactéries *E*. *Coli,* à raison d'une bactérie par microcuvette.

Le dispositif miniature conforme à l'Invention ainsi préparé peut ensuite être utilisé dans diverses applications biologiques.

### EXEMPLE 2 : ISOLEMENT ET FIXATION DE BACTÉRIES SUR UN DISPOSITIF MINIATURE SOUS L'ACTION D'UN CHAMP ÉLECTRIQUE

### 1) Fixation des bactéries par champ électrique

On prépare une suspension de bactéries *E. Coli* DH5α dans de l'eau permutée à raison de 10⁹ bactéries/ml.

Un dispositif miniature identique à celui utilisé ci-dessus à l'exemple 1 est ensuite immergé dans cette suspension de bactéries.

On applique ensuite pendant 10 secondes, un champ électrique entre les deux électrodes de la puce : potentiel de + 0,9 V sur l'électrode où l'on désire fixer la bactérie, le potentiel de l'autre électrode étant fixé à - 2V.

Lorsque la fixation est réalisée, le dispositif est alors rincé à l'eau et séché à la soufflette d'azote.

On obtient un dispositif sur lequel sont immobilisées des bactéries E. *Coli*, à raison d'une bactérie par microcuvette.

Le dispositif miniature conforme à l'Invention ainsi préparé peut ensuite être utilisé dans diverses applications biologiques.

### EXEMPLE 3 : PRÉPARATION D'UNE PUCE A ADN PAR PCR A PARTIR D'UN DISPOSITIF MINIATURE CONFORME A L'INVENTION

Cet exemple décrit le protocole général de préparation d'une puce à ADN sur un dispositif miniature conforme à l'Invention.

### 1) Dépôt d'une amorce sens sur un dispositif miniature conforme à l'Invention

On prépare une solution à 2,3⁻⁴ M d'une amorce sens modifiée en position 5' par un groupement pyrrole (0,77 µM) dans du perchlorate de lithium à 2,3⁻² M.

Cette solution est déposée sur le dispositif miniature conforme à l'Invention et tel que préparé ci-dessus à l'exemple 2.

On applique ensuite pendant 3 secondes, un champ électrique entre les deux électrodes de la puce : potentiel de + 2,9 V appliqué sur l'électrode où l'on désire fixer l'amorce, l'autre électrode étant mise à la masse.

Le dispositif miniature est ensuite rincé à l'eau et séché à la soufflette d'azote.

### 2) Lyse des bactéries

Cette étape est réalisée par chauffage des bactéries à une température de 94°C pendant 2 minutes.

### 3) Réalisation de la PCR

La PCR est réalisée en utilisant la solution suivante : Tris-HCL 1 mM, KCl 5 mM, MgCl₂ 2 mM, dNTP 0,8 mM ; amorce anti-sens marquée par la biotine en position 5' : 0,1 µM, BSA 1 mg/mL, Taq DNA polymérase de chez ROCHE à 0,02 unités/µl et amorce sens à 0,01 µM.

La puce est ensuite immergée dans l'huile.

La PCR est réalisée dans les conditions suivantes : 3 minutes à 94°C puis 30 cycles à 94°C pendant 30 secondes, 60°C pendant 30 secondes et 72°C pendant 1 minute et 30 secondes ; puis 72°C pendant 3 minutes et enfin 25°C pendant 30 secondes. Les cycles sont effectués dans un thermocycleur Hybaid.

Le dispositif miniature est ensuite rincé à l'eau après la fin des cycles PCR.

L'ADN amplifié est marqué par fluorescence par de la Streptavidine phycoérythrine.

La visualisation de la fluorescence est ensuite effectuée à l'aide d'un microscope à fluorescence.

### EXEMPLE 4 : EXPRESSION D'UN MATÉRIEL GÉNÉTIQUE SUR UN DISPOSITIF MINIATURE CONFORME A L'INVENTION

Dans cet exemple, il a été utilisé un dispositif miniature identique à celui utilisé dans les exemples 1 à 3 ci-dessus et comportant 8100 puits.

Des bactéries *E coli* renfermant le gène *bla* de la Beta-lactamase TEM-1 ont été immobilisées sur ce dispositif selon le procédé d'immobilisation décrit ci-dessus à l'exemple 2.

Le gène *bla* a ensuite été amplifié par PCR dans les conditions décrites ci-dessus à l'exemple 3.

Le produit PCR ainsi obtenu comporte (de la position 5' vers la position 3') un promoteur T7, un site de fixation des ribosomes et le cadre ouvert de lecture du gène *bla.*

Une puce témoin destinée à servir de contrôle a été réalisée en amplifiant de la manière le gène GFP de la protéine verte fluorescente ("Green Fluorescent Protein").

Afin de permettre l'expression des protéines, ces deux puces ont été recouvertes d'un mélange de réaction de transcription/traduction *in vitro* couplé.

Ce mélange a été préparé selon la méthode telle que décrite par Pratt et Nevin (Pratt J.M., "Coupled transcription-translation in prokaryotic cell-free systems, Transcription and Translation : A practical approach", Hames B.D. & Higgins S.J., JLR Press, 1984, 179-209 ; Nevin D.E. and Pratt J.M., "A Coupled in vitro transcription-translation system for the exclusive synthesis of polypeptides expressed from the T7 promotor, FEBS, 1991, 291, 2, 259-263).

Chaque puce a ensuite été recouverte d'une lamelle de verre et mise dans une cloche à vide pour permettre le remplissage efficace de chaque microréservoir par dégazage.

Les puces ainsi préparées ont été incubées en atmosphère saturée en eau (pour limiter l'évaporation des réactifs) pendant 90 minutes à 30°C. les lamelles de verre ont ensuite été enlevées et chaque puce a été recouverte de nitrocéphine (Oxoid, réf. SR112C). Ce réactif a pour propriété de virer du jaune au rouge lorsqu'il est hydrolysé par une beta-lactamase.

Les résultats obtenus (non représentés) sont les suivants :
Sur la puce contrôle (gène de la GFP), le réactif est resté jaune.
Sur la puce comportant le produit PCR du gène *bla,* le réactif a viré au rouge, indiquant que la beta-lactame TEM-1 a été exprimée sur cette puce.

## Revendications

1. Utilisation d'un dispositif miniature de séparation et/ou d'isolement d'objets biologiques, comportant au moins une première électrode intégrée au dispositif et au moins une deuxième électrode intégrée ou externe au dispositif, constitué par une structure pourvue d'une matrice de microcuvettes réactionnelles, chaque microcuvette comportant un fond constituant une zone de réception, **caractérisé en ce que** ledit fond est dépourvu de trou et que la surface maximale dudit fond de chaque microcuvette est définie de manière à isoler un objet biologique unique, ladite structure étant reliée à un circuit d'alimentation pour créer une différence de potentiel entre ladite première électrode et ladite deuxième électrode, pour des applications liées à la biologie moléculaire.

2. Utilisation selon la revendication 1, pour la fabrication de puces à acides nucléiques (puces à ADN), la fabrication de puces à protéines, le tri de banques génomiques, l'analyse de banques de transcrits, la mesure d'une variation de l'activité d'une protéine fonctionnelle, la réalisation d'antivirogrammes et pour le criblage de protéines ou le criblage pharmaceutique.

3. Utilisation selon la revendication 1 ou 2, **caractérisée par le fait que** la surface maximale du fond de chaque microcuvette est inférieure ou égale à deux fois la plus petite surface de l'objet biologique à isoler.

4. Utilisation selon la revendication 3, **caractérisée par le fait que** la surface dudit fond est inférieure ou égale à la plus petite surface de l'objet biologique à isoler.

5. Utilisation selon l'une quelconque des revendications précédentes, **caractérisée par le fait que** la surface maximale du fond de chaque microcuvette est comprise entre 1 µm² et 400 µm².

6. Utilisation selon la revendication 5, **caractérisée par le fait que** la surface maximale du fond de chaque microcuvette est comprise entre 1 et 50 µm².

7. Utilisation selon l'une quelconque des revendications précédentes, **caractérisée par le fait que** la matrice de microcuvettes réactionnelles du dispositif est surmontée au moins en partie d'une ou plusieurs couches de matériaux isolants et/ou d'une grille en plastique biocompatible rapportée, de façon à former une matrice de microréservoirs.

8. Utilisation selon la revendication 7, **caractérisée par le fait que** les matériaux isolants sont choisis parmi les polyimides et les résines.

9. Utilisation selon l'une quelconque des revendications précédentes, **caractérisée par le fait que** la longueur et/ou la largeur des microréservoirs est comprise entre 5 et 500 µm.

10. Utilisation selon l'une quelconque des revendications 7 à 9, **caractérisée par le fait que** le dispositif comprend au moins deux couches de matériaux isolants, et qu'une desdites couches ne fait pas partie intégrante du dispositif mais se présente sous la forme d'une pièce rapportée amovible venant recouvrir au moins en partie ledit dispositif.

11. Utilisation selon l'une quelconque des revendications précédentes, **caractérisée par le fait qu'**une face de la première électrode intégrée au dispositif constitue le fond des microcuvettes.

12. Utilisation selon l'une quelconque des revendications 1 à 10, **caractérisée par le fait que** le fond des microcuvettes est constitué par une couche en verre, en plastique ou en silicium.

13. Utilisation selon l'une quelconque des revendications précédentes, **caractérisée par le fait que** la deuxième électrode est intégrée au dispositif et qu'elle est située dans un plan espacé du fond des microcuvettes.

14. Utilisation selon l'une quelconque des revendications 1 à 12, **caractérisée par le fait que** la deuxième électrode est externe au dispositif et qu'elle est solidaire d'un capot ou d'un couvercle.

15. Utilisation selon l'une quelconque des revendications précédentes, **caractérisée par le fait qu'**un réactif apte à fixer l'objet biologique à isoler est fixé sur au moins une partie de la zone de réception des microcuvettes réactionnelles.

16. Utilisation selon la revendication 15, prise en combinaison avec l'une quelconque des revendications 11, 13 ou 14, **caractérisée par le fait que** le réactif est choisi parmi les copolymères conducteurs sur lesquels sont fixés des protéines, des peptides ou toutes molécules spécifiques du type d'objet biologique à fixer.

17. Utilisation selon la revendication 16, **caractérisée par le fait que** les copolymères conducteurs sont choisis parmi les polypyrroles.

18. Utilisation selon la revendication 16 ou 17, **caractérisée par le fait que** le réactif est un copolymère pyrrole-biotine-streptavidine-biotine-molécule spécifique.

19. Utilisation selon la revendication 15, prise en combinaison avec l'une quelconque des revendications 12 à 14, **caractérisée par le fait que** le réactif est choisi parmi des polymères non spécifiques du type d'objet biologique à fixer.

20. Utilisation selon la revendication 19, **caractérisée par le fait que** lesdits polymères sont de la poly-L-lysine.

21. Utilisation selon la revendication 15, prise en combinaison avec l'une quelconque des revendications 12 à 14, **caractérisée par le fait que** le réactif est une protéine ou un peptide et que ladite couche en verre, en plastique ou en silicium est recouverte d'une couche de silane modifié par des fonctions -NHS ou aldéhyde sur lesquelles est fixé ledit réactif.

22. Utilisation selon l'une quelconque des revendications précédentes, **caractérisée par le fait que** le dispositif est équipé d'un moyen de fermeture.

23. Utilisation selon l'une quelconque des revendications précédentes, **caractérisée par le fait que** les objets biologiques à isoler sont des cellules procaryotes ou eucaryotes, des virus, des liposomes ou des microalgues.

24. Utilisation selon l'une quelconque des revendications précédentes, **caractérisée par le fait que** les objets biologiques sont choisis parmi les bactéries issues d'une banque de séquences génomiques.

25. Procédé de séparation et/ou d'isolement d'objets biologiques pour l'obtention de puces à acides nucléiques ou à protéines ou pour la détection de protéines fonctionnelles, **caractérisé en ce qu'**il comprend au moins les étapes a) et b) suivantes :
a) la mise en contact d'au moins un dispositif miniature de séparation et/ou d'isolement d'objets biologiques, comportant au moins une première électrode intégrée au dispositif et au moins une deuxième électrode intégrée ou externe au dispositif, constitué par une structure pourvue d'une matrice de microcuvettes réactionnelles, chaque microcuvette comportant un fond dépourvu de trou et constituant une zone de réception, la surface maximale dudit fond de chaque microcuvette étant définie de manière à isoler un objet biologique unique, ladite structure étant reliée à un circuit d'alimentation pour créer une différence de potentiel entre ladite première électrode et ladite deuxième électrode, avec une solution d'objets biologiques homogénéisée, pour permettre la fixation desdits objets biologiques au fond des microcuvettes sur les zones de réception, à raison d'au plus un objet biologique par microcuvette,
b) l'élimination des objets biologiques non fixés de façon à obtenir un dispositif miniature sur lequel les objets biologiques à isoler sont immobilisés.

26. Procédé selon la revendication 25, **caractérisé par le fait que** la fixation des objets biologiques est réalisée par l'intermédiaire d'un champ électrique.

27. Procédé selon la revendication 25, **caractérisé par le fait que** la fixation des objets biologiques est réalisée par l'intermédiaire d'un réactif fixé sur au moins une partie du fond des microcuvettes réactionnelles.

28. Procédé selon l'une quelconque des revendications 25 à 27, **caractérisé en ce qu'**il comporte une étape préliminaire à l'étape a) consistant à transformer les objets biologiques par un vecteur de clonage portant un gène qui code pour une protéine spécifique qui sera présente en surface des objets biologiques de telle sorte que la fixation des objets biologiques se fera par une interaction entre cette protéine et un réactif spécifique de ladite protéine et/ou par clonage d'un fragment d'ADN dans un vecteur, ledit fragment empêchant l'expression d'un gène codant pour une protéine toxique pour l'objet biologique recombinant.

29. Procédé selon l'une quelconque des revendications 25 à 28, **caractérisé en ce que** l'on utilise un dispositif comportant des microréservoirs et qu'il comporte une étape au cours de laquelle le matériel génétique des objets biologiques est libéré dans lesdits microréservoirs.

30. Procédé selon l'une quelconque des revendications 25 à 28, **caractérisé en ce que** l'on utilise un dispositif comportant des microréservoirs et une deuxième électrode interne ou externe adaptée à la perméabilisation des objets biologiques fixés.

31. Procédé selon l'une quelconque des revendications 25 à 30, **caractérisé en ce qu'**il est utilisé pour l'obtention de puces à acides nucléiques et qu'il comporte une étape au cours de laquelle le matériel génétique des objets isolés est amplifié pour obtenir des séquences amplifiées.

32. Procédé selon la revendication 31, **caractérisé en ce que** les séquences amplifiées sont fixées par électropolymérisation sur une deuxième électrode du dispositif utilisé.

33. Procédé selon l'une quelconque des revendications 25 à 30, **caractérisé en ce qu'**il est utilisé pour l'obtention de puces à protéines ou pour la détection de protéines fonctionnelles et qu'il comprend, après l'étape b), une étape de transcription et de traduction du matériel génétique des objets biologiques isolés en protéines.

34. Procédé selon la revendication 33, **caractérisé en ce qu'**il comprend une étape de fixation desdites protéines sur une deuxième électrode du dispositif utilisé.

35. Procédé selon la revendication 33 ou 34, **caractérisé en ce qu'**il est utilisé pour la détection de protéines fonctionnelles et qu'il comprend, après l'étape de traduction, une étape de révélation d'au moins une propriété des protéines isolées.

36. Procédé selon la revendication 33 ou 34, **caractérisé en ce qu'**il comprend une étape de mesure de la variation de l'activité desdites protéines fonctionnelles consistant à tester l'effet de différentes molécules sur l'activité desdites protéines fonctionnelles.

37. Procédé selon l'une quelconque des revendications 33 à 35, **caractérisé en ce qu'**il est utilisé pour l'obtention de puces à protéines recombinantes et qu'il comporte, avant l'étape a), une étape de transformation des objets biologiques par un vecteur de clonage afin de permettre le marquage des protéines recombinantes par un épitope universel.

38. Procédé d'analyse d'une banque de transcrit **caractérisé en ce qu'**il comprend au moins les étapes a) et b) telles que définies à la revendication 25 ainsi qu'une étape de détection d'un gène ayant subi une différence d'expression.

## Claims

1. The use of a miniature device for separating and/or isolating biological objects, comprising at least one first electrode integrated into the device and at least one second electrode integrated into or external to the device, consisting of a structure provided with a matrix of reaction microcuvettes, each microcuvette comprising a bottom constituting a reception zone, **characterized in that** said bottom is devoid of holes and **in that** the maximum surface area of said bottom of each microcuvette is defined so as to isolate a single biological object, said structure being connected to a supply circuit so as to create a potential difference between said first electrode and said second electrode, for applications related to molecular biology.

2. The use as claimed in claim 1, for producing nucleic acid chips (DNA chips), producing protein chips, sorting genomic libraries, analyzing transcript libraries, measuring a variation in the activity of a functional protein, effecting antivirograms, and for protein screening or pharmaceutical screening.

3. The use as claimed in claim 1 or 2, **characterized in that** the maximum surface area of the bottom of each microcuvette is less than or equal to twice the smallest surface area of the biological object to be isolated.

4. The use as claimed in claim 3, **characterized in that** the surface area of said bottom is less than or equal to the smallest surface area of the biological object to be isolated.

5. The use as claimed in any one of the preceding claims, **characterized in that** the maximum surface area of the bottom of each microcuvette is between 1 µm² and 400 µm².

6. The use as claimed in claim 5, **characterized in that** the maximum surface area of the bottom of each microcuvette is between 1 and 50 µm².

7. The use as claimed in any one of the preceding claims, **characterized in that** the matrix of reaction microcuvettes of the device is surmounted, at le ast in part, by one or more layers of isolating materials and/or by an attached grid made of biocompatible plastic, so as to form a matrix of microreservoirs.

8. The use as claimed in claim 7, **characterized in that** the isolating materials are chosen from polyimides and resins.

9. The use as claimed in any one of the preceding claims, **characterized in that** the microreservoirs are between 5 and 500 µm in length and/or in width.

10. The use as claimed in any one of claims 7 to 9, **characterized in that** the device comprises at least two layers of isolating materials, and **in that** one of said layers is not an integral part of the device but is in the form of a removable, mounted component which covers at least in part said device.

11. The use as claimed in any one of the preceding claims, **characterized in that** one face of the first electrode integrated into the device constitutes the bottom of the microcuvettes.

12. The use as claimed in any one of claims 1 to 10, **characterized in that** the bottom of the microcuvettes consists of a layer made of glass, plastic or silicon.

13. The use as claimed in any one of the preceding claims, **characterized in that** the second electrode is integrated into the device and **in that** it is located in a plane apart from the bottom of the microcuvettes.

14. The use as claimed in any one of claims 1 to 12, **characterized in that** the second electrode is external to the device and **in that** it is joined to a cap or a lid.

15. The use as claimed in any one of the preceding claims, **characterized in that** a reagent capable of attaching the biological object to be isolated is attached to at least one part of the reception zone of the reaction microcuvettes.

16. The use as claimed in claim 15, taken in combination with any one of claims 11, 13 or 14, **characterized in that** the reagent is chosen from conducting copolymers to which are attached proteins, peptides or any molecules specific to the type of biological object to be attached.

17. The use as claimed in claim 16, **characterized in that** the conducting copolymers are chosen from polypyrroles.

18. The use as claimed in claim 16 or 17, **characterized in that** the reagent is a pyrrole-biotin-streptavidin-biotin-specific molecule copolymer.

19. The use as claimed in claim 15, taken in combination with any one of claims 12 to 14, **characterized in that** the reagent is chosen from polymers not specific for the type of biological object to be attached.

20. The use as claimed in claim 19, **characterized in that** said polymers are poly-L-lysine.

21. The use as claimed in claim 15, taken in combination with any one of claims 12 to 14, **characterized in that** the reagent is a protein or a peptide and **in that** said layer made of glass, plastic or silicon is covered with a layer of silane modified by -NHS or aldehyde functions to which said reagent is attached.

22. The use as claimed in any one of the preceding claims, **characterized in that** the device is equipped with a closing means.

23. The use as claimed in any one of the preceding claims, **characterized in that** the biological objects to be isolated are prokaryotic or eukaryotic cells, viruses, liposomes and microalgae.

24. The use as claimed in any one of the preceding claims, **characterized in that** the biological objects are chosen from bacteria derived from a library of genomic sequences.

25. A method for separating and/or isolating biological objects for obtaining nucleic acid chips or protein chips or for detecting functional proteins, **characterized in that** it comprises at least the following steps a) and b):
a) bringing at least one miniature device for separating and/or isolating biological objects, comprising at least one first electrode integrated into the device and at least one second electrode integrated into or external to the device, consisting of a structure provided with a matrix of reaction microcuvettes, each microcuvette comprising a bottom devoid of holes and constituting a reception zone, the maximum surface area of said bottom of each microcuvette being defined so as to isolate a single biological object, said structure being connected to a supply circuit so as to create a potential difference between said first electrode and said second electrode, into contact with a homogenized solution of biological objects, so as to allow the attachment of said biological objects at the bottom of the microcuvettes, on the reception zones, at a rate of at most one biological object per microcuvette,
b) removing the unattached biological objects so as to obtain a miniature device on which the biological objects to be isolated are immobilized.

26. The method as claimed in claim 25, **characterized in that** the biological objects are attached via an electric field.

27. The method as claimed in claim 25, **characterized in that** the biological objects are attached via a reagent attached to at least one part of the bottom of the reaction microcuvettes.

28. The method as claimed in any one of claims 25 to 27, **characterized in that** it comprises a step which is a step preliminary to step a), consisting in transforming the biological objects with a cloning vector bearing a gene which encodes a specific protein which will be present at the surface of the biological objects such that the attachment of the biological objects will take place via an interaction between this protein and a reagent specific for said protein, and/or by cloning a DNA fragment into a vector, said fragment preventing the expression of a gene encoding a protein which is toxic for the recombinant biological object.

29. The method as claimed in any one of claims 25 to 28, **characterized in that** a device comprising microreservoirs is used and **in that** it comprises a step during which the genetic material of the biological objects is released into said microreservoirs.

30. The method as claimed in any one of claims 25 to 28, **characterized in that** a device comprising microreservoirs and an internal or external second electrode suitable for permeabilization of the attached biological objects is used.

31. The method as claimed in any one of claims 25 to 30, **characterized in that** it is used for obtaining nucleic acid chips and **in that** it comprises a step during which the genetic material of the isolated objects is amplified so as to obtain amplified sequences.

32. The method as claimed in claim 31, **characterized in that** the amplified sequences are attached by electropolymerization to a second electrode of the device used.

33. The method as claimed in any one of claims 25 to 30, **characterized in that** it is used for obtaining protein chips or for detecting functional proteins, and **in that** it comprises, after step b), a step for transcription and translation of the genetic material of the isolated biological objects into proteins.

34. The method as claimed in claim 33, **characterized in that** it comprises a step of attachment of said proteins to a second electrode of the device used.

35. The method as claimed in claim 33 or 34, **characterized in that** it is used for detecting functional proteins and **in that** it comprises, after the translation step, a step for revealing at least one property of the isolated proteins.

36. The method as claimed in claim 33 or 34, **characterized in that** it comprises a step for measuring the variation in the activity of said functional proteins, consisting in assaying the effect of various molecules on the activity of said functional proteins.

37. The method as claimed in any one of claims 33 to 35, **characterized in that** it is used for obtaining recombinant protein chips and **in that** it comprises, before step a), a step for transforming the biological objects with a cloning vector in order to enable the recombinant proteins to be labeled with a universal epitope.

38. A method for analyzing a transcript library, **characterized in that** it comprises at least steps a) and b) as defined in claim 25, and also a step for detecting a gene which has experienced a difference in expression.

## Patentansprüche

1. Verwendung einer Miniaturvorrichtung zur Abtrennung und/oder Isolierung von biologischen Targets, umfassend wenigstens eine erste in die Vorrichtung integrierte Elektrode und wenigstens eine zweite Elektrode, die in die Vorrichtung integriert ist oder außerhalb der Vorrichtung ist, die durch eine Struktur gebildet wird, welche mit einer Matrix von Reaktionsmikroküvetten ausgestattet ist, wobei jede Mikroküvette einen Boden umfasst, der eine Aufnahmezone bildet, **dadurch gekennzeichnet, dass** der Boden ohne Loch ist und dass die maximale Oberfläche des Bodens jeder Küvette zur Isolierung eines einzigen biologischen Targets definiert ist, wobei die Struktur mit einem Speisestromkreis verbunden ist, um eine Potentialdifferenz zwischen der ersten Elektrode und der zweiten Elektrode zu erzeugen, für Anwendungen, die mit der Molekularbiologie in Verbindung stehen.

2. Verwendung nach Anspruch 1 für die Herstellung von Nucleinsäurechips (DNA-Chips), die Herstellung von Proteinchips, das Sortieren von Genbanken, die Analyse von Transkriptbanken, das Messen einer Veränderung der Aktivität eines funktionellen Proteins, die Verwirklichung von Antivirogrammen und für das Screening von Proteinen oder das pharmazeutische Screening.

3. Verwendung nach Anspruch 1 oder 2, **dadurch gekennzeichnet, dass** die maximale Oberfläche des Bodens jeder Mikroküvette unter dem zweifachen der kleinsten Oberfläche des zu isolierenden biologischen Targets ist oder gleich dem zweifachen der kleinsten Oberfläche des zu isolierenden biologischen Targets ist.

4. Verwendung nach Anspruch 3, **dadurch gekennzeichnet, dass** die Oberfläche des Bodens unter der kleinsten Oberfläche des zu isolierenden biologischen Targets ist oder gleich der kleinsten Oberfläche des zu isolierenden biologischen Targets ist.

5. Verwendung nach einem der vorangehenden Ansprüche, **dadurch gekennzeichnet, dass** die maximale Oberfläche des Bodens jeder Mikroküvette zwischen 1 µm² und 400 µm² liegt.

6. Verwendung nach Anspruch 5, **dadurch gekennzeichnet, dass** die maximale Oberfläche des Bodens jeder Mikroküvette zwischen 1 und 50 µm² liegt.

7. Verwendung nach einem der vorangehenden Ansprüche, **dadurch gekennzeichnet, dass** die Matrix von Reaktionsmikroküvetten der Vorrichtung wenigstens teilweise von einer oder mehreren Schichten aus isolierenden Materialien und/oder einem aufgesetzten Gitter aus biokompatiblem Kunststoff überragt wird, so dass eine Matrix mit Mikroreservoiren gebildet wird.

8. Verwendung nach Anspruch 7, **dadurch gekennzeichnet, dass** die isolierenden Materialien aus Polyimiden und Harzen ausgewählt sind.

9. Verwendung nach einem der vorangehenden Ansprüche, **dadurch gekennzeichnet, dass** die Länge und/oder die Breite der Mikroreservoire zwischen 5 und 500 µm liegt.

10. Verwendung nach einem der Ansprüche 7 bis 9, **dadurch gekennzeichnet, dass** die Vorrichtung wenigstens zwei Schichten aus isolierenden Materialien umfasst und dass eine der Schichten kein integraler Teil der Vorrichtung ist, sich aber in Form eines abnehmbaren aufgesetzten Stücks präsentiert, das die Vorrichtung wenigstens teilweise bedeckt.

11. Verwendung nach einem der vorangehenden Ansprüche, **dadurch gekennzeichnet, dass** eine Seite der ersten Elektrode, die in die Vorrichtung integriert ist, den Boden der Mikroküvetten bildet.

12. Verwendung nach einem der Ansprüche 1 bis 10, **dadurch gekennzeichnet, dass** der Boden der Mikroküvetten durch eine Schicht aus Glas, Kunststoff oder Silicium gebildet wird.

13. Verwendung nach einem der vorangehenden Ansprüche, **dadurch gekennzeichnet, dass** die zweite Elektrode in die Vorrichtung integriert ist und dass sie sich in einer Ebene befindet, die vom Boden der Mikroküvetten beabstandet ist.

14. Verwendung nach einem der Ansprüche 1 bis 12, **dadurch gekennzeichnet, dass** die zweite Elektrode außerhalb der Vorrichtung ist und dass sie aus einem Stück mit einer Haube oder einem Deckel ist.

15. Verwendung nach einem der vorangehenden Ansprüche, **dadurch gekennzeichnet, dass** ein Reagens, das fähig ist, das biologische Target zu isolieren, an wenigstens einem Teil der Aufnahmezone der Reaktionsmikroküvetten fixiert ist.

16. Verwendung nach Anspruch 15 in Kombination mit einem der Ansprüche 11, 13 oder 14, **dadurch gekennzeichnet, dass** das Reagens unter den leitenden Copolymeren ausgewählt ist, an denen Proteine, Peptide oder alle Moleküle, die für den Typ des zu fixierenden biologischen Targets spezifisch sind, fixiert werden.

17. Verwendung nach Anspruch 16, **dadurch gekennzeichnet, dass** die leitenden Copolymere unter Polypyrrolen ausgewählt sind.

18. Verwendung nach Anspruch 16 oder 17, **dadurch gekennzeichnet, dass** das Reagens ein Copolymer ist, das für das Pyrrol-Biotin-Streptavidin-Biotin-Molekül spezifisch ist.

19. Verwendung nach Anspruch 15 in Kombination mit einem der Ansprüche 12 bis 14, **dadurch gekennzeichnet, dass** das Reagens unter Polymeren ausgewählt ist, die für den Typ des zu fixierenden biologischen Targets nicht spezifisch sind.

20. Verwendung nach Anspruch 19, **dadurch gekennzeichnet, dass** die Polymere Poly-L-Lysin sind.

21. Verwendung nach Anspruch 15 in Kombination mit einem der Ansprüche 12 bis 14, **dadurch gekennzeichnet, dass** das Reagens ein Protein oder ein Peptid ist und dass die Schicht aus Glas, Kunststoff oder Silicium von einer Schicht aus Silan, modifiziert durch -NHS- oder Aldehyd-Funktionen, bedeckt ist, an denen das Reagens fixiert ist.

22. Verwendung nach einem der vorangehenden Ansprüche, **dadurch gekennzeichnet, dass** die Vorrichtung mit einem Schließmittel ausgestattet ist.

23. Verwendung nach einem der vorangehenden Ansprüche, **dadurch gekennzeichnet, dass** die zu isolierenden biologischen Targets Prokaryoten- oder Eukaryotenzellen, Viren, Liposome oder Mikroalgen sind.

24. Verwendung nach einem der vorangehenden Ansprüche, **dadurch gekennzeichnet, dass** die biologischen Targets unter den Bakterien, die aus einer Bank für genomische Sequenzen stammen, ausgewählt sind.

25. Verfahren zur Abtrennung und/oder Isolierung von biologischen Targets zum Erhalt von Nucleinsäurechips oder Proteinchips oder für die Detektion von funktionellen Proteinen, **dadurch gekennzeichnet, dass** es wenigstens die folgenden Stufen a) und b) umfasst:
a) Inkontaktbringen wenigstens einer Miniaturvorrichtung zur Abtrennung und/oder Isolierung von biologischen Targets, umfassend wenigstens eine erste in die Vorrichtung integrierte Elektrode und wenigstens eine zweite Elektrode, die in die Vorrichtung integriert ist oder außerhalb der Vorrichtung ist, die durch eine Struktur gebildet wird, welche mit einer Matrix von Reaktionsmikroküvetten ausgestattet ist, wobei jede Mikroküvette einen Boden ohne Loch, der eine Aufnahmezone bildet, umfasst, wobei die maximale Oberfläche des Bodens jeder Mikroküvette zur Isolierung eines einzigen biologischen Targets definiert ist, wobei die Struktur mit einem Speisestromkreis verbunden ist, um eine Potentialdifferenz zwischen der ersten Elektrode und der zweiten Elektrode zu erzeugen, mit einer homogenisierten Lösung von biologischen Targets, um die Fixierung der biologischen Targets am Boden der Mikroküvetten an den Aufnahmezonen, höchstens ein biologisches Target pro Mikroküvette, zu erlauben,
b) Eliminierung der nicht-fixierten biologischen Targets derart, dass eine Miniaturvorrichtung erhalten wird, an der die zu isolierenden biologischen Targets immobilisiert sind.

26. Verfahren nach Anspruch 25, **dadurch gekennzeichnet, dass** die Fixierung der biologischen Targets mit Hilfe eines elektrischen Feldes durchgeführt wird.

27. Verfahren nach Anspruch 25, **dadurch gekennzeichnet, dass** die Fixierung der biologischen Targets über ein Reagens, das an wenigstens einem Teil des Bodens der Reaktionsmikroküvetten fixiert ist, durchgeführt wird.

28. Verfahren nach einem der Ansprüche 25 bis 27, **dadurch gekennzeichnet, dass** es eine der Stufe a) vorgeschaltete Stufe umfasst, die darin besteht, die biologischen Targets mit einem Klonierungsvektor zu transformieren, welcher ein Gen trägt, das für ein spezifisches Protein codiert, das sich an der Oberfläche der biologischen Targets präsentieren wird, derart, dass die Fixierung der biologischen Targets durch Wechselwirkung zwischen diesem Protein und einem für das genannte Protein spezifischen Reagens erfolgen wird, und/oder durch Klonierung eines DNA-Fragments in einen Vektor zu transformieren, wobei das Fragment die Expression eines Gens, das für ein für das rekombinante biologische Target toxische Protein codiert, verhindert.

29. Verfahren nach einem der Ansprüche 25 bis 28, **dadurch gekennzeichnet, dass** man eine Vorrichtung verwendet, die Mikroreservoire umfasst, und dass es eine Stufe umfasst, in deren Verlauf das genetische Material der biologischen Targets in die Mikroreservoire freigesetzt wird.

30. Verfahren nach einem der Ansprüche 25 bis 28, **dadurch gekennzeichnet, dass** man eine Vorrichtung verwendet, die Mikroreservoire und eine zweite innere oder äußere Elektrode, angepasst an die Permeabilisierung der fixierten biologischen Targets, umfasst.

31. Verfahren nach einem der Ansprüche 25 bis 30, **dadurch gekennzeichnet, dass** es für die Herstellung von Nucleinsäurechips verwendet wird und dass es eine Stufe umfasst, in deren Verlauf das genetische Material der isolierten Targets amplifiziert wird, um amplifizierte Sequenzen zu erhalten.

32. Verfahren nach Anspruch 31, **dadurch gekennzeichnet, dass** die amplifizierten Sequenzen durch Elektropolymerisation auf einer zweiten Elektrode der verwendeten Vorrichtung fixiert werden.

33. Verfahren nach einem der Ansprüche 25 bis 30, **dadurch gekennzeichnet, dass** es für die Herstellung von Proteinchips oder für die Detektion von funktionellen Proteinen verwendet wird und dass es nach der Stufe b) eine Stufe der Transkription und Translation des genetischen Materials der isolierten biologischen Targets in Proteine umfasst.

34. Verfahren nach Anspruch 33, **dadurch gekennzeichnet, dass** es eine Stufe der Fixierung der Proteine an einer zweiten Elektrode der verwendeten Vorrichtung umfasst.

35. Verfahren nach Anspruch 33 oder 34, **dadurch gekennzeichnet, dass** es für die Detektion von funktionellen Proteinen verwendet wird und dass es nach der Translationsstufe eine Stufe der Entwicklung wenigstens einer Eigenschaft der isolierten Proteine umfasst.

36. Verfahren nach Anspruch 33 oder 34, **dadurch gekennzeichnet, dass** es eine Stufe der Messung der Veränderung der Aktivität der genannten funktionellen Proteine umfasst, die darin besteht, die Wirkung von verschiedenen Molekülen auf die Aktivität der funktionellen Proteine zu untersuchen.

37. Verfahren nach einem der Ansprüche 33 bis 35, **dadurch gekennzeichnet, dass** es für die Herstellung von Chips rekombinanter Proteine verwendet wird und dass es vor der Stufe a) eine Stufe der Transformation der biologischen Targets durch einen Klonierungsvektor umfasst, um so die Markierung der rekombinanten Proteine durch ein universelles Epitop zu ermöglichen.

38. Verfahren zur Analyse einer Transkriptbank, **dadurch gekennzeichnet, dass** es wenigstens die Stufen a) und b), wie sie in Anspruch 25 definiert sind, sowie eine Stufe zur Detektion eines Gens, das eine Expressionsänderung erfahren hat, umfasst.
